# NEW EUROPEAN PATENT SPECIFICATION

(11) **EP 1 181 366 B2**
(45) Date of publication and mention of the opposition decision: **21.08.2013**
(45) Mention of the grant of the patent: 04.03.2009
(21) Application number: 00937943.9
(22) Date of filing: 30.05.2000
(51) Int. Cl.: C12N 15/12, C07K 14/715, C07K 16/28, A61K 38/17

(54) **MAMMALIAN RECEPTOR PROTEINS; RELATED REAGENTS AND METHODS**
SÄUGETIER-REZEPTORPROTEINE; ÄHNLICHE REAGENTIEN UND METHODEN
PROTEINES DE RECEPTEUR MAMMALIENNES, REACTIFS ET PROCEDES Y RELATIFS

(30) Priority: 01.06.1999 US 322913
(43) Date of publication of application: 27.02.2002
(60) Divisional application: 08103122.1
(73) Proprietor: Merck Sharp & Dohme Corp., Rahway, NJ 07065-0907 (US)
(72) Inventor: DOWLING, Lynette, M., Redwood City, CA 94062 (US); TIMANS, Jacqueline, C., Mountain View, CA 94043 (US); GORMAN, Daniel, M., Palo Alto, CA 94306 (US); KASTELEIN, Robert, A., Portola Valley, CA 94028 (US); BAZAN, Fernando, J., Menlo Park, CA 94025 (US)
(74) Representative: Horgan, James Michael Frederic
(86) International application number: PCT/US2000/014867
(87) International publication number: WO 2000/073451

(56) References cited:
- WO-A-99/20755
- US-A- 5 716 804
- DATABASE SWISSPROT [Online] Sequence P40190: Interleukin-6 receptor beta chain precursor, membrane glycoprotein GP130 from rat; 1 February 1995 (1995-02-01) XP002152341
- WANG YANG; NESBITT JAMES E; FUENTES NELSON L; FULLER GERALD M: "Molecular cloning and characterization of the rat liver IL-6 signal transducing molecule, gp130" GENOMICS, vol. 14, no. 3, 1992, pages 666-672,

## Description

### FIELD OF THE INVENTION

The present invention relates to compositions and methods for affecting mammalian physiology, including immune system function. In particular, it provides methods to regulate development and/or the immune system. Diagnostic and therapeutic uses of these materials are also disclosed.

### BACKGROUND OF THE INVENTION

Recombinant DNA technology refers generally to techniques of integrating genetic information from a donor source into vectors for subsequent processing, such as through introduction into a host, whereby the transferred genetic information is copied and/or expressed in the new environment. Commonly, the genetic information exists in the form of complementary DNA (cDNA) derived from messenger RNA (mRNA) coding for a desired protein product. The carrier is frequently a plasmid having the capacity to incorporate cDNA for later replication in a host and, in some cases, actually to control expression of the cDNA and thereby direct synthesis of the encoded product in the host. See, e.g., Sambrook, et al. (1989) Molecular Cloning: A Laboratory Manual, (2d ed.) vols. 1-3, CSH Press, NY.

For some time, it has been known that the mammalian immune response is based on a series of complex cellular interactions, called the "immune network". Recent research has provided new insights into the inner workings of this network. While it remains clear that much of the immune response does, in fact, revolve around the network-like interactions of lymphocytes, macrophages, granulocytes, and other cells, immunologists now generally hold the opinion that soluble proteins, known as lymphokines, cytokines, or monokines, play critical roles in controlling these cellular interactions. Thus, there is considerable interest in the isolation, characterization, and mechanisms of action of cell modulatory factors, an understanding of which will lead to significant advancements in the diagnosis and therapy of numerous medical abnormalities, e.g., immune system disorders.

Lymphokines apparently mediate cellular activities in a variety of ways. See, e.g., Paul (ed. 1996) Fundamental Immunology 3d ed., Raven Press, New York; and Thomson (ed. 1994) The Cytokine Handbook 2d ed., Academic Press, San Diego. They have been shown to support the proliferation, growth, and/or differentiation of pluripotential hematopoietic stem cells into vast numbers of progenitors comprising diverse cellular lineages which make up a complex immune system. Proper and balanced interactions between the cellular components are necessary for a healthy immune response. The different cellular lineages often respond in a different manner when lymphokines are administered in conjunction with other agents.

Cell lineages especially important to the immune response include two classes of lymphocytes: B-cells, which can produce and secrete immunoglobulins (proteins with the capability of recognizing and binding to foreign matter to effect its removal), and T-cells of various subsets that secrete lymphokines and induce or suppress the B-cells and various other cells (including other T-cells) making up the immune network. These lymphocytes interact with many other cell types.

Research to better understand and treat various immune disorders has been hampered by the general inability to maintain cells of the immune system in vitro. Immunologists have discovered that culturing many of these cells can be accomplished through the use of T-cell and other cell supernatants; which contain various growth factors, including many of the lymphokines.

Various growth and regulatory factors exist which modulate morphogenetic development. This includes, e.g., the Toll ligands, which signal through binding to receptors which share structural, and mechanistic, features characteristic of the IL-1 receptors. See, e.g., Lemaitre, et al. (1996) Cell 86:973-983; and Belvin and Anderson (1996) Ann. Rev. Cell & Devel. Biol. 12:393-416. Other receptors for cytokines are also known. Often, there are at least two critical subunits in the functional receptor. See, e.g., Gonda and D'Andrea (1997) Blood 89:355-369; Presky, et al. (1996) Proc. Nat'l Acad. Sci. USA 93:14002-14007; Drachman and Kaushansky (1995) Curr. Opin. Hematol. 2:22-28; Theze (1994) Eur. Cytokine Netw. 5:353-368; and Lemmon and Schlessinger (1994) Trends Biochem. Sci. 19:459-463. WO 99/20755 discloses a murine GP130 (mGP130) sequence.

From the foregoing, it is evident that the discovery and development of new soluble proteins and their receptors, including ones similar to lymphokines, should contribute to new therapies for a wide range of degenerative or abnormal conditions which directly or indirectly involve development, differentiation or function, e.g., of the immune system and/or hematopoietic cells. In particular, the discovery and understanding of novel receptors for lymphokine-like molecules which enhance or potentiate the beneficial activities of other lymphokines would be highly advantageous. The present invention provides new receptors for ligands exhibiting similarity to cytokine like compositions and related compounds, and methods for their use.

### SUMMARY OF THE INVENTION

The present invention is directed to novel receptors related to cytokine receptors, e.g., primate, cytokine receptor like molecular structures, designated DNAX Cytokine Receptor Subunits (DCRS), and their biological activities. In particular, it provides description of one subunit, designated DCRS2. It includes nucleic acids coding for the polypeptides themselves and methods for their production and use. The nucleic acids of the invention are characterized, in part, by their homology to cloned complementary DNA (cDNA) sequences enclosed herein.

The present invention provides an isolated polypeptide comprising at least 70% amino acid sequence homology to the mature amino acid sequence (residues 1-361) of SEQ ID NO: 2. Insofar as they are encompassed by the appendant claims, the following embodiments are provided, namely a composition of matter selected from: a substantially pure or recombinant DCRS2 polypeptide comprising at least three distinct nonoverlapping segments of at least four amino acids identical to segments of SEQ ID NO: 2; a substantially pure or recombinant DCRS2 polypeptide comprising at least two distinct nonoverlapping segments of at least five amino acids identical to segments of SEQ ID NO: 2; a natural sequence DCRS2 comprising mature SEQ ID NO: 2; or a fusion polypeptide comprising DCRS2 sequence. In certain embodiment, the invention embraces such a substantially pure or isolated antigenic DCRS2 polypeptide, wherein the distinct nonoverlapping segments of identity: include one of at least eight amino acids; include one of at least four amino acids and a second of at least five amino acids; include at least three segments of at least four, five, and six amino acids, or include one of at least twelve amino acids. Other embodiments include wherein the: DCRS2 polypeptide: comprises a mature sequence of Table 1; is an unglycosylated form of DCRS2; is from a primate, such as a human; comprises at least seventeen amino acids of SEQ ID NO: 2; exhibits at least four nonoverlapping segments of at least seven amino acids of SEQ ID NO: 2; is a natural allelic variant of DCRS2; has a length at least about 30 amino acids; exhibits at least two nonoverlapping epitopes which are specific for a primate DCRS2; is glycosylated; has a molecular weight of at least 30 kD with natural glycosylation; is a synthetic polypeptide; is attached to a solid substrate; is conjugated to another chemical moiety; is a 5-fold or less substitution from natural sequence; or is a deletion or insertion variant from a natural sequence. Still other embodiments include a composition comprising: a substantially pure DCRS2 and another Interferon Receptor family member; a sterile DCRS2 polypeptide; the DCRS2 polypeptide and a carrier, wherein the carrier is: an aqueous compound, including water, saline, and/or buffer; and/or formulated for oral, rectal, nasal, topical, or parenteral administration. Fusion polypeptide embodiments include those comprising: mature protein sequence of Table 1; a detection or purification tag, including a FLAG, His6, or Ig sequence; or sequence of another interferon receptor protein. Kit embodiments include those comprising such a polypeptide, and: a compartment comprising the protein or polypeptide; or instructions for use or disposal of reagents in the kit.

Binding compound embodiments include, e.g., a binding compound comprising an antigen binding site from an antibody, which specifically binds to a natural DCRS2 polypeptide, wherein: the binding compound is in a container; the DCRS2 polypeptide is from a human; the binding compound is an Fv, Fab, or Fab2 fragment; the binding compound is conjugated to another chemical moiety; or the antibody: is raised against a peptide sequence of a mature polypeptide of Table 1; is raised against a mature DCRS2; is raised to a purified human DCRS2; is immunoselected; is a polyclonal antibody; binds to a denatured DCRS2; exhibits a Kd to antigen of at least 30 µM; is attached to a solid substrate, including a bead or plastic membrane; is in a sterile composition; or is detectably labeled, including a radioactive or fluorescent label. Kits include those comprising the binding compound, and: a compartment comprising the binding compound; or instructions for use or disposal of reagents in the kit.

Methods are provided, e.g., of producing an antigen:antibody complex, comprising contacting under appropriate conditions a primate DCRS2 polypeptide of the invention with a described antibody, thereby allowing the complex to form. This includes wherein: the complex is purified from other interferon receptors; the complex is purified from other antibody; the contacting is with a sample comprising an interferon; the contacting allows quantitative detection of the antigen; the contacting is with a sample comprising the antibody; or the contacting allows quantitative detection of the antibody.

Various related compositions are provided, e.g., a composition comprising: a sterile binding compound, as described, or the described binding compound and a carrier, wherein the carrier is: an aqueous compound, including water, saline, and/or buffer; and/or formulated for oral, rectal, nasal, topical, or parenteral administration.

Nucleic acid embodiments include, e.g., an isolated or recombinant nucleic acid encoding the DCRS2 polypeptide wherein said DCRS2 polypeptide comprises at least 70% amino acid sequence homology to the mature amino acid sequence (residues 1-361) of SEQ ID NO: 2. Insofar as they are encompassed by the appendant claims, the following embodiments are also provided, namely wherein the: DCRS2 is from a human; or the nucleic acid: encodes an antigenic peptide sequence of Table 1; encodes a plurality of antigenic peptide sequences of Table 1; exhibits identity over at least thirteen nucleotides to a natural cDNA encoding the segment; is an expression vector; further comprises an origin of replication; is from a natural source; comprises a detectable label; comprises synthetic nucleotide sequence; is less than 6 kb, preferably less than 3 kb; is from a primate; comprises a natural full length coding sequence; is a hybridization probe for a gene encoding the DCRS2; or is a PCR primer, PCR product, or mutagenesis primer. Other embodiments of the invention include a cell or tissue comprising the described recombinant nucleic acid. Preferably, the cell is: a prokaryotic cell; a eukaryotic cell; a bacterial cell; a yeast cell; an insect cell; a mammalian cell; a mouse cell; a primate cell; or a human cell.

Kit embodiments include those comprising a described nucleic acid, and: a compartment comprising the nucleic acid; a compartment further comprising a primate DCRS2 polypeptide; or instructions for use or disposal of reagents in the kit.

Alternative nucleic acids disclosed herein include a nucleic acid which: hybridizes under wash conditions of 30 minutes at 30°C and less than 2M salt to the coding portion of SEQ ID NO: 1; or exhibits identity over a stretch of at least about 30 nucleotides to a primate DCRS2. Certain embodiments include those wherein the nucleic acid hybridizes to the coding portion of SEQ ID NO: 1 and the wash conditions are at 45°C and/or 500 mM salt; or the wash conditions are at 55°C and/or 150 mM salt. Also disclosed herein, the stretch is at least 55 nucleotides; or the stretch is at least 75 nucleotides.

Other uses include those of modulating physiology or development of a cell or tissue culture cells comprising contacting the cell with an agonist or antagonist of a mammalian DCRS2. Preferably, the cell is transformed with a nucleic acid encoding a DCRS2 and another cytokine receptor subunit.

### DETAILED DESCRIPTION OF THE PREFERRED EMBODIMENTS

### OUTLINE

I. General
II. Activities
III. Nucleic acids
   A. encoding fragments, sequence, probes
   B. mutations, chimeras, fusions
   C. making nucleic acids
   D. vectors, cells comprising
IV. Proteins, Peptides
   A. fragments, sequence, immunogens, antigens
   B. muteins
   C. agonists/antagonists, functional equivalents
   D. making proteins
V. Making nucleic acids, proteins
   A. synthetic
   B. recombinant
   C. natural sources
VI. Antibodies
   A. polyclonals
   B. monoclonal
   C. fragments; Kd
   D. anti-idiotypic antibodies
   E. hybridoma cell lines
VII. Kits and Methods to quantify DCRS2
   A. ELISA
   B. assay mRNA encoding
   C. qualitative/quantitative
   D. kits
VIII. Therapeutic compositions, methods
   A. combination compositions
   B. unit dose
   C. administration
IX. Screening
X. Ligands

### I. General

The present invention provides the amino acid sequence and DNA sequence of mammalian, herein primate, cytokine receptor-like subunit molecules, this one designated DNAX Cytokine Receptor Subunit 2 (DCRS2) having particular defined properties, both structural and biological. Various cDNAs encoding these molecules were obtained from primate, e.g., human, cDNA sequence libraries. Other primate or other mammalian counterparts would also be desired.

Some of the standard methods applicable are described or referenced, e.g., in Maniatis, et al. (1982) Molecular cloning, A Laboratory Manual, Cold Spring Harbor Laboratory, Cold Spring Harbor Press; Sambrook, et al. (1989) Molecular Cloning: A Laboratory Manual, (2d ed.), vols. 1-3, CSH Press, NY; Ausubel, et al., Biology, Greene Publishing Associates, Brooklyn, NY; or Ausubel, et al. (1987 and periodic supplements) Current Protocols in Molecular Biology, Greene/Wiley, New York.

Nucleotide (SEQ ID NO: 1) and corresponding amino acid sequence (SEQ ID NO: 2) of a human DCRS2 coding segment is shown in Table 1. It is likely that there is at least one splice variant with a longer intracellular domain, and will probably exhibit characteristic signaling motifs. The predicted signal sequence is indicated, but may depend on cell type, or may be a few residues in either direction. Potential N glycosylation sites are at Asparagine residues 6, 24, 58, 118, 157, 209, and 250. Disulfide linkages are likely to be found between cysteine residues at positions 29 and 78; and a conserved C_CXW motif is found at positions 110/121/123. The tryptophan at 219; and the WxxWS motif from 281-285 are notable. The segment from about 1-101 is an Ig domain; from about 102-195 is a cytokine binding domain 1; from about 196-297 is a cytokine binding domain 2; from about 298-330 is a linker; from about 331-353 is a transmembrane segment; and from about 354-361 is an intracellular domain. These sites and boundaries are notable.'

The reverse translation nucleic acid sequence is provided in Table 2.

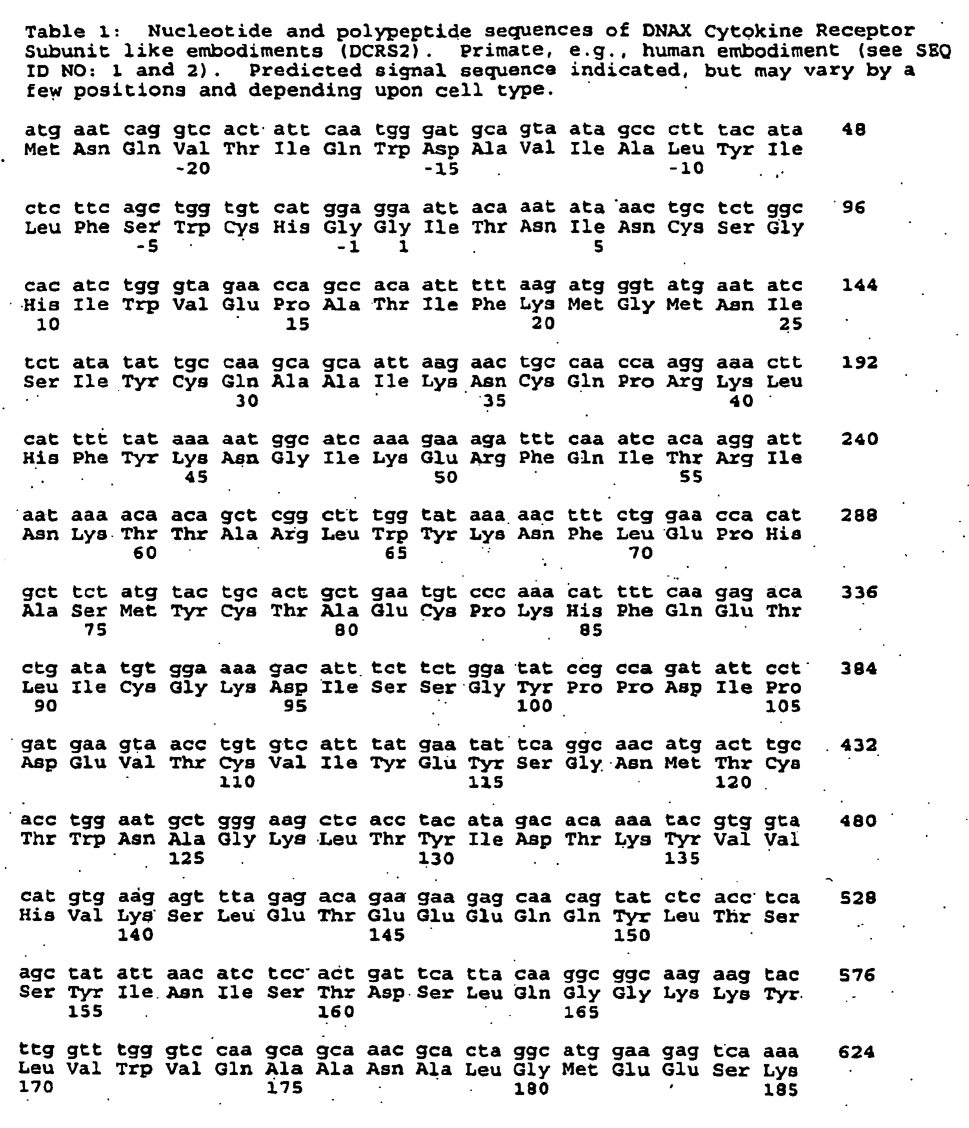

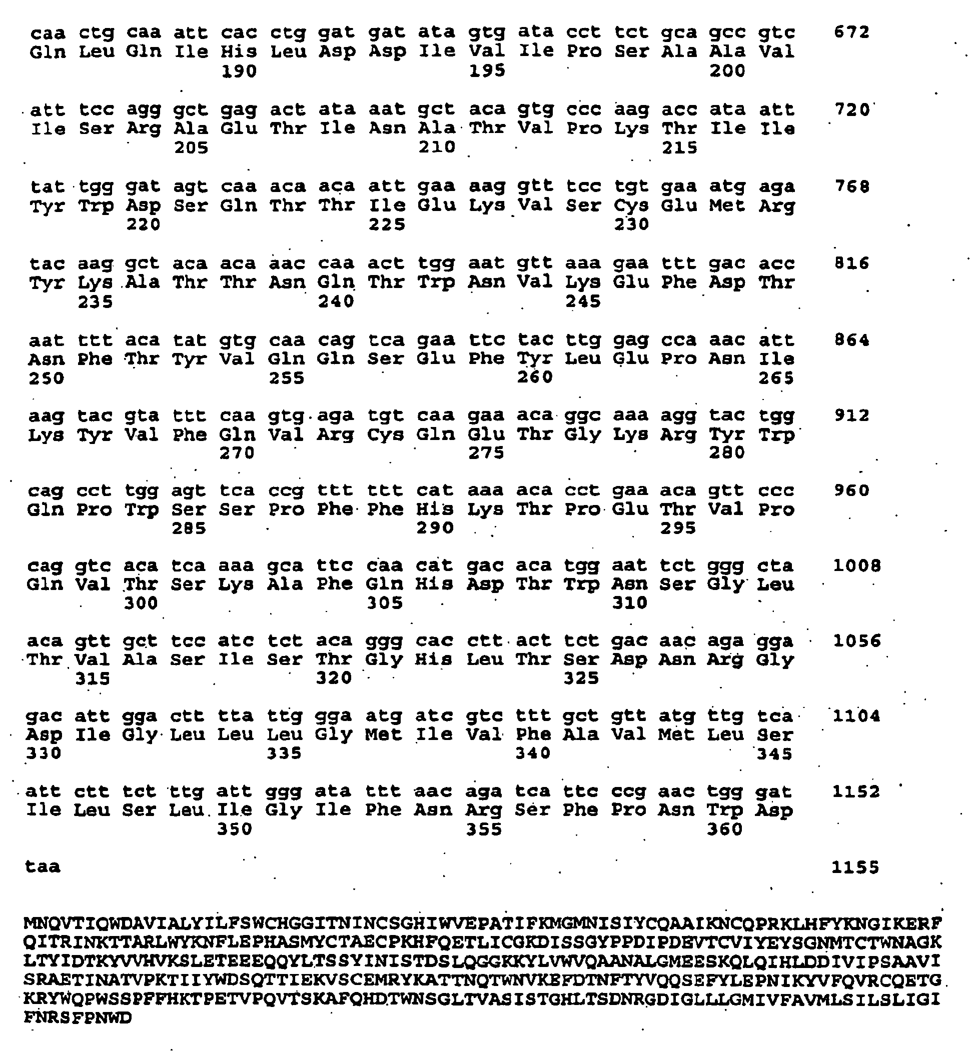

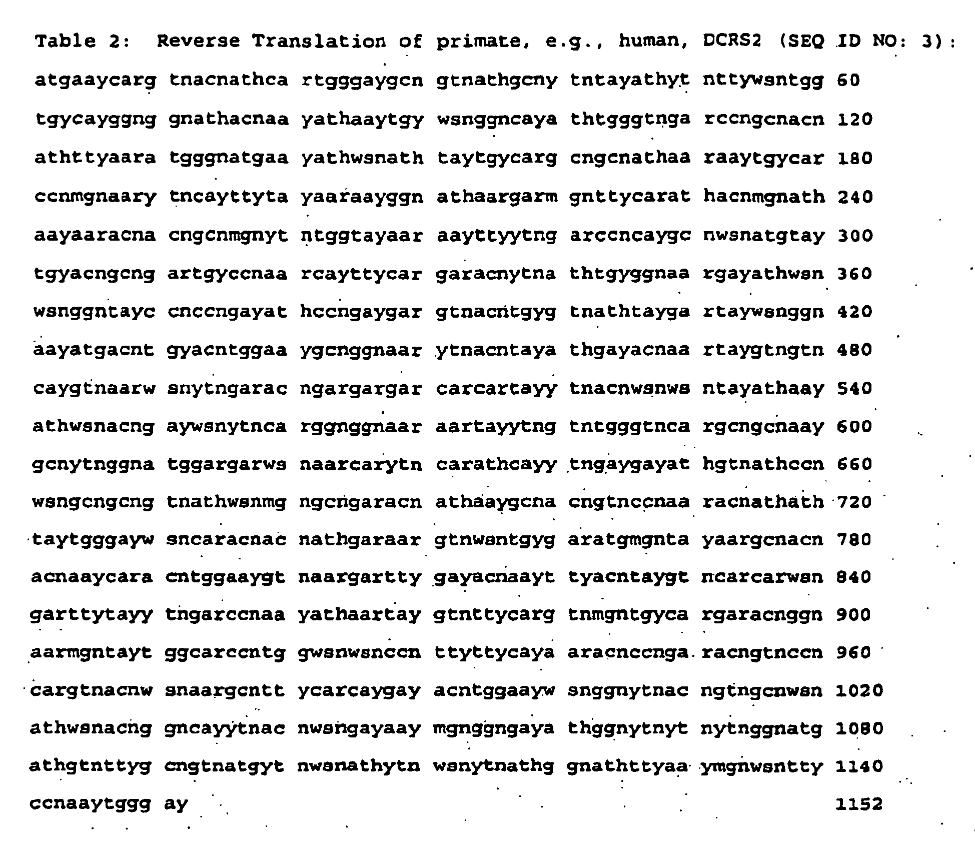

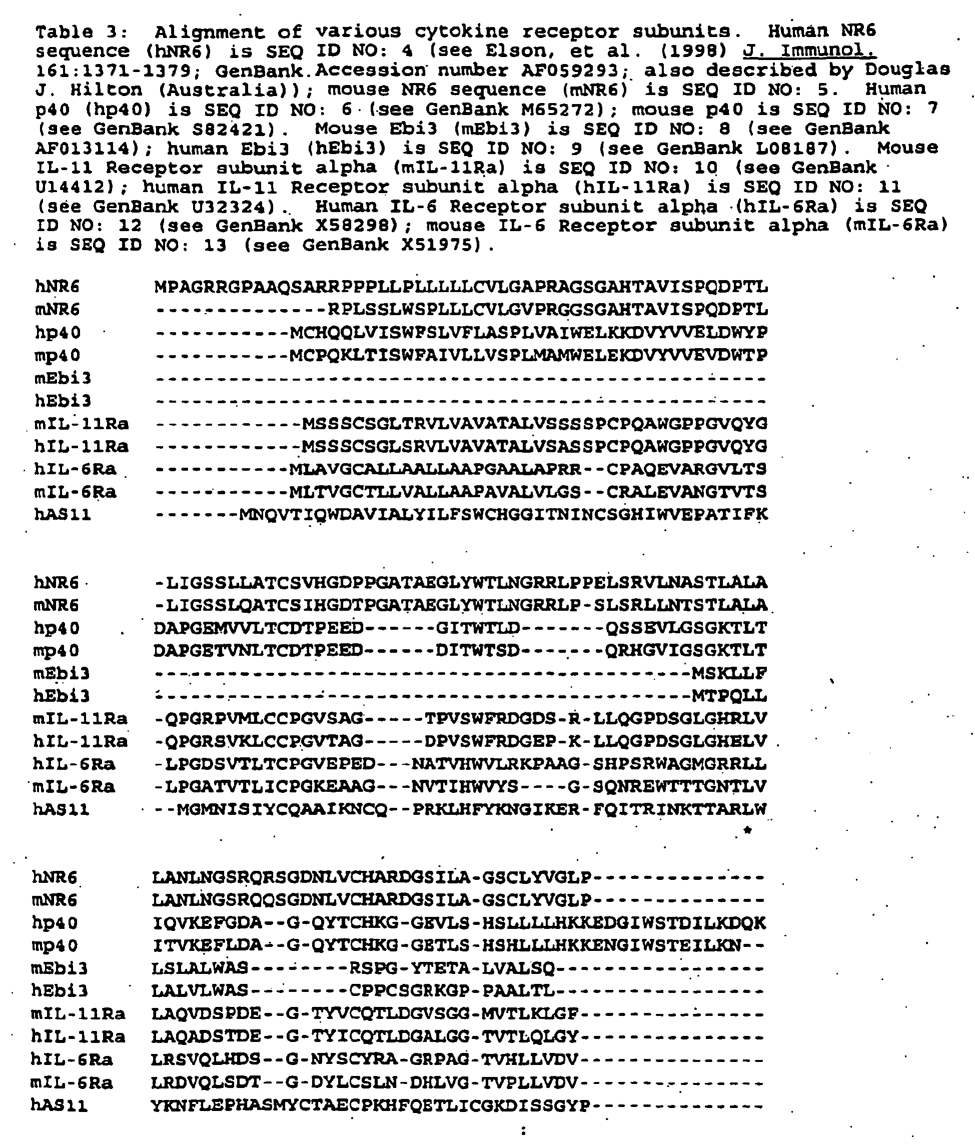

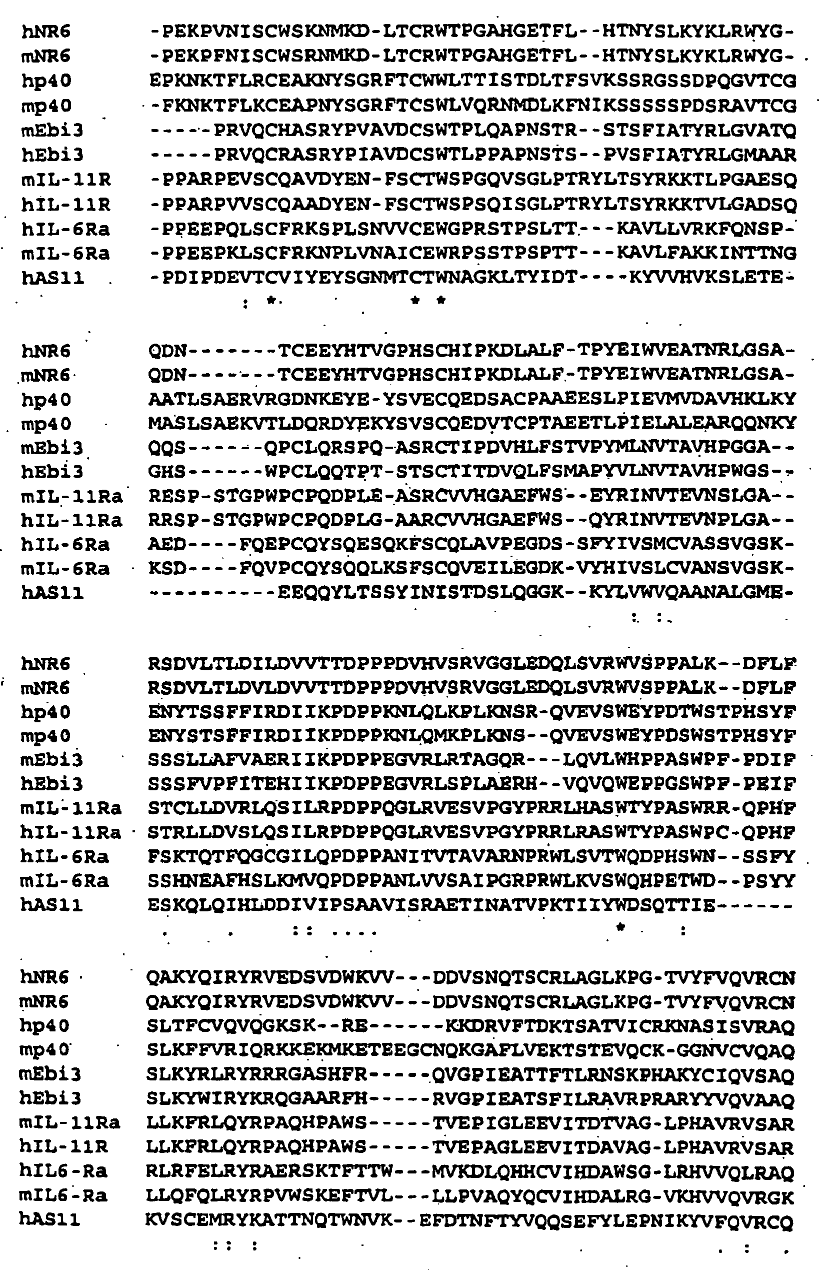

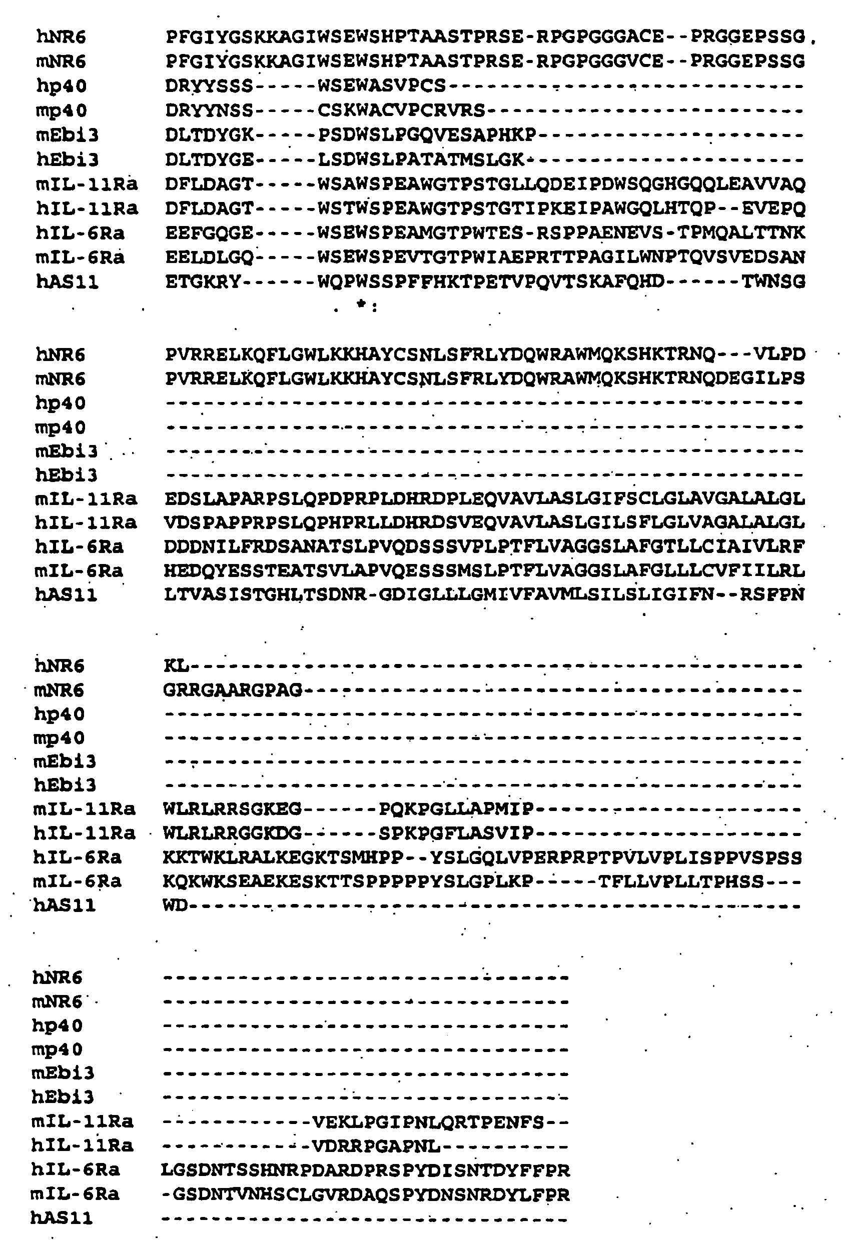

Table 3 shows comparison of the available sequences of primate and rodent receptors with the primate, e.g., human DCRS2 (AS11). The DCRS2 shows particular similarity to the IL-11 receptor subunit alpha, though it may be aligned with the p40 and IL-6 receptor alpha subunits. It is likely an alpha subunit, and thus should bind to ligand without the need for a beta subunit. The biology is likely to be similar to the IL-6 receptor subunit.

As used herein, the term DCRS2 shall be used to describe a protein comprising the amino acid sequence shown in Table 1. In many cases, a substantial fragment thereof will be functionally or structurally equivalent, including, e.g., an extracellular or intracellular domain. The invention also includes a protein variation of the respective DCRS2 allele whose sequence is provided, e.g., a mutein or soluble extracellular construct. Typically, such agonists or antagonists will exhibit less than about 10% sequence differences, and thus will often have between 1- and 11-fold substitutions, e.g., 2-, 3-, 5-, 7-fold, and others. It also encompasses allelic and other variants, e.g., natural polymorphic, of the protein described. Typically, it will bind to its corresponding biological ligand, perhaps in a dimerized state with an alpha receptor subunit, with high affinity, e.g., at least about 100 nM, usually better than about 30 nM, preferably better than about 10 nM, and more preferably at better than about 3 nM. The term shall also be used herein to refer to related naturally occurring forms, e.g., alleles, polymorphic variants, and metabolic variants of the mammalian protein. Preferred forms of the receptor complexes will bind the appropriate ligand with an affinity and selectivity appropriate for a ligand-receptor interaction.

This invention also encompasses combinations of proteins or peptides having substantial amino acid sequence identity with the amino acid sequence in Table 1. It will include sequence variants with relatively few substitutions, e.g., preferably less than about 3-5.

A substantial polypeptide "fragment", or "segment", is a stretch of amino acid residues of at least about 8 amino acids, amino acids, often at least 14 amino acids, more often at least 16 amino acids, typically at least 18 amino acids, more typically at least 20 amino acids, usually at least 22 amino acids, more usually at least 24 amino acids, preferably at least 26 amino acids, more preferably at least 28 amino acids, and at least about 30 and, in particularly preferred embodiments, at least about 30 or more amino acids. Sequences of segments of different proteins can be compared to one another over appropriate length stretches. In many situations, fragments may exhibit functional properties of the intact subunits, e.g., the extracellular domain of the transmembrane receptor may retain the ligand binding features, and may be used to prepare a soluble receptor-like complex.

Amino acid sequence homology, or sequence identity, is determined by optimizing residue matches. In some comparisons, gaps may be introduces, as required. See, e.g., Needleham, et al., (1970) J. Mol. Biol. 48:443-453; Sankoff, et al., (1983) chapter one in Time Warps, String Edits, and Macromolecules: The Theory and Practice of Sequence Comparison. Addison-Wesley, Reading, MA; and software packages from IntelliGenetics, Mountain View, CA; and the University of Wisconsin Genetics Computer Group (GCG), Madison, WI. This changes when considering conservative substitutions as matches. Conservative substitutions typically include substitutions within the following groups: glycine, alanine; valine, isoleucine, leucine; aspartic acid, glutamic acid; asparagine, glutamine; serine, threonine; lysine, arginine; and phenylalanine, tyrosine. Homologous amino acid sequences are intended to include natural allelic and interspecies variations in the cytokine sequence. Typical homologous proteins or peptides will have from 50-100% homology (if gaps can be introduced), to 60-100% homology (if conservative substitutions are included) with an amino acid sequence segment of Table 1. For the polypeptides of the invention, homology measures will be at least 70%, generally at least 76%, more generally at least 81%, often at least 85%, more often at least 88%, typically at least 90%, more typically at least 92%, usually at least 94%, more usually at least 95%, preferably at least 96%, and more preferably at least 97%, and in particularly preferred embodiments, at least 98% or more. The degree of homology will vary with the length of the compared segments. Homologous proteins or peptides, such as the allelic variants, will share most biological activities with the embodiments described in Table 1.

As used herein, the term "biological activity" is used to describe, without limitation, effects on inflammatory responses, innate immunity, and/or morphogenic development by cytokine-like ligands. For example, these receptors should mediate phosphatase or phosphorylase activities, which activities are easily measured by standard procedures. See, e.g., Hardie, et al. (eds. 1995) The Protein Kinase FactBook vols. I and II, Academic Press, San Diego, CA; Hanks, et al. (1991) Meth. Enzymol. 200:38-62; Hunter, et al. (1992) Cell 70:375-388; Lewin (1990) Cell 61:743-752; Pines, et al. (1991) Cold Spring Harbor Symp. Quant. Biol. 56:449-463; and Parker, et al. (1993) Nature 363:736-738. The receptors, or portions thereof, may be useful as phosphate labeling enzymes to label general or specific substrates. The subunits may also be functional immunogens to elicit recognizing antibodies, or antigens capable of binding antibodies.

The terms ligand, agonist, antagonist, and analog of, e.g., a DCRS2, include molecules that modulate the characteristic cellular responses to cytokine ligand proteins, as well as molecules possessing the more standard structural binding competition features of ligand-receptor interactions, e.g., where the receptor is a natural receptor or an antibody. The cellular responses likely are typically mediated through receptor tyrosine kinase pathways.

Also, a ligand is a molecule which serves either as a natural ligand to which said receptor, or an analog thereof, binds, or a molecule which is a functional analog of the natural ligand. The functional analog may be a ligand with structural modifications, or may be a wholly unrelated molecule which has a molecular shape which interacts with the appropriate ligand binding determinants. The ligands may serve as agonists or antagonists, see, e.g., Goodman, et al. (eds. 1990) Goodman & Gilman's: The Pharmacological Bases of Therapeutics, Pergamon Press, New York.

Rational drug design may also be based upon structural studies of the molecular shapes of a receptor or antibody and other effectors or ligands. See, e.g., Herz, et al. (1997) J. Recept. Signal Transduct. Res. 17:671-776; and Chaiken, et al. (1996) Trends Biotechnol. 14:369-375. Effectors may be other proteins which mediate other functions in response to ligand binding, or other proteins which normally interact with the receptor. One means for determining which sites interact with specific other proteins is a physical structure determination, e.g., x-ray crystallography or 2 dimensional NMR techniques. These will provide guidance as to which amino acid residues form molecular contact regions. For a detailed description of protein structural determination, see, e.g., Blundell and Johnson (1976) Protein Crystallography, Academic Press, New York.

### II. Activities

The cytokine receptor-like proteins will have a number of different biological activities, e.g., modulating cell proliferation, or in phosphate metabolism, being added to or removed from specific substrates, typically proteins. Such will generally result in modulation of an inflammatory function, other innate immunity response, or a morphological effect. The subunit will probably have a specific low affinity binding to the ligand.

The DCRS2 has the characteristic motifs of a receptor signaling through the JAK pathway. See, e.g., Ihle, et al. (1997) Stem Cells 15 (suppl. 1) :105-111; Silvennoinen, et al. (1997) APMIS 105:497-509; Levy (1997) Cytokine Growth Factor Review 8:81-90; Winston and Hunter (1996) Current Biol. 6:668-671; Barrett (1996) Baillieres Clin. Gastroenterol. 10:1-15; and Briscoe, et al. (1996) Philos. Trans. R. Soc. Lond. B. Biol. Sci. 351:167-171.

The biological activities of the cytokine receptor subunits will be related to addition or removal of phosphate moieties to substrates, typically in a specific manner, but occasionally in a non specific manner. Substrates may be identified, or conditions for enzymatic activity may be assayed by standard methods, e.g., as described in Hardie, et al. (eds. 1995) The Protein Kinase FactBook vols. I and II, Academic Press, San Diego, CA; Hanks, et al. (1991) Meth. Enzymol. 200:38-62; Hunter, et al. (1992) Cell 70:375-388; Lewin (1990) Cell 61:743-752; Pines, et al. (1991) Cold Spring Harbor Symp. Quant. Biol, 56:449-463; and Parker, et al. (1993) Nature 363:736-738.

The receptor subunits may combine to form functional complexes, e.g., which may be useful for binding ligand or preparing antibodies. These will have substantial diagnostic uses, including detection or quantitation.

### III. Nucleic Acids

This invention contemplates use of isolated nucleic acid or fragments, e.g., which encode these or closely related proteins, or fragments thereof, e.g., to encode a corresponding polypeptide, preferably one which is biologically active. In addition, this invention covers isolated or recombinant DNAs which encode combinations of such proteins or polypeptides having characteristic sequences, e.g., of the DCRS2s, insofar as these are encompassed by the appendant claims. Typically, the nucleic acid is capable of hybridizing, under appropriate conditions, with a nucleic acid sequence segment shown in Table 1, but preferably not with a corresponding segment of other receptors described in Table 3. Said biologically active protein or polypeptide can be a full length protein, or fragment, and will typically have a segment of amino acid sequence highly homologous, e.g., exhibiting significant stretches of identity, to one shown in Table 1. Further, this invention covers the use of isolated or recombinant nucleic acid, or fragments thereof, which encode proteins having fragments which are equivalent to the DCRS2 proteins insofar as these are encompassed by the appendant claims. The isolated nucleic acids can have the respective regulatory sequences in the 5' and 3' flanks, e.g., promoters, enhancers, poly-A addition signals, and others from the natural gene. Combinations, as described, are also provided.

An "isolated" nucleic acid is a nucleic acid, e.g., an RNA, DNA, or a mixed polymer, which is substantially pure, e.g., separated from other components which naturally accompany a native sequence, such as ribosomes, polymerases, and flanking genomic sequences from the originating species. The term embraces a nucleic acid sequence which has been removed from its naturally occurring environment, and includes recombinant or cloned DNA isolates, which are thereby distinguishable from naturally occurring compositions, and chemically synthesized analogs or analogs biologically synthesized by heterologous systems. A substantially pure molecule includes isolated forms of the molecule, either completely or substantially pure.

An isolated nucleic acid will generally be a homogeneous composition of molecules, but will, in some embodiments, contain heterogeneity, preferably minor. This heterogeneity is typically found at the polymer ends or portions not critical to a desired biological function or activity.

A "recombinant" nucleic acid is typically defined either by its method of production or its structure. In reference to its method of production, e.g., a product made by a process, the process is use of recombinant nucleic acid techniques, e.g., involving human intervention in the nucleotide sequence. Typically this intervention involves in vitro manipulation, although under certain circumstances it may involve more classical animal breeding techniques. Alternatively, it can be a nucleic acid made by generating a sequence comprising fusion of two fragments which are not naturally contiguous to each other, but is meant to exclude products of nature, e.g., naturally occurring mutants as found in their natural state. Thus, for example, products made by transforming cells with an unnaturally occurring vector is encompassed, as are nucleic acids comprising sequence derived using any synthetic oligonucleotide process. Such a process is often done to replace a codon with a redundant codon encoding the same or a conservative amino acid, while typically introducing or removing a restriction enzyme sequence recognition site. Alternatively, the process is performed to join together nucleic acid segments of desired functions to generate a single genetic entity comprising a desired combination of functions not found in the commonly available natural forms, e.g., encoding a fusion protein. Restriction enzyme recognition sites are often the target of such artificial manipulations, but other site specific targets, e.g., promoters, DNA replication sites, regulation sequences, control sequences, or other useful features may be incorporated by design. A similar concept is intended for a recombinant, e.g., fusion, polypeptide. This will include a dimeric repeat. Specifically included, insofar as they are encompassed by the appendant claims, are synthetic nucleic acids which, by genetic code redundancy, encode equivalent polypeptides to fragments of DCRS2 and fusions of sequences from various different related molecules, e.g., other cytokine receptor family members.

A "fragment" in a nucleic acid context is a contiguous segment of at least about 17 nucleotides, generally at least 21 nucleotides, more generally at least 25 nucleotides, ordinarily at least 30 nucleotides, more ordinarily at least 35 nucleotides, often at least 39 nucleotides, more often at least 45 nucleotides, typically at least 50 nucleotides, more typically at least 55 nucleotides, usually at least 60 nucleotides, more usually at least 66 nucleotides, usually at least 72 nucleotides, more usually at least 79 nucleotides, and in particular instances will be at least 85 or more nucleotides. Typically, fragments of different genetic sequences can be compared to one another over appropriate length stretches, particularly defined segments such as the domains described below.

A nucleic acid which codes for the DCRS2 will be particularly useful to identify genes, mRNA, and cDNA species which code for itself or closely related proteins, as well as DNAs which code for polymorphic, allelic, or other genetic variants, e.g., from different individuals or related species. Preferred probes for such screens are those regions of the interleukin which are conserved between different polymorphic variants or which contain nucleotides which lack specificity, and will preferably be full length or nearly so. In other situations, polymorphic variant specific sequences will be more useful.

This invention further covers, insofar as they are encompassed by the appendant claims, recombinant nucleic acid molecules and fragments having a nucleic acid sequence identical to or highly homologous to the isolated DNA set forth herein. In particular, the sequences will often be operably linked to DNA segments which control transcription, translation, and DNA replication. These additional segments typically assist in expression of the desired nucleic acid segment.

Homologous, or highly identical, nucleic acid sequences, when compared to one another; e.g., DCRS2 sequences, exhibit significant similarity. The standards for homology in nucleic acids are either measures for homology generally used in the art by sequence comparison or based upon hybridization conditions. Comparative hybridization conditions are described in greater detail below.

Substantial identity in the nucleic acid sequence comparison context means either that the segments or their complementary strands, when compared, are identical when optimally aligned, with appropriate nucleotide insertions or deletions, in at least about 60% of the nucleotides, generally at least 66%, and in the invention: ordinarily at least 71%, often at least 76%, more often at least 80%, usually at least 84%, more usually at least 88%, typically at least 91%, more typically at least about 93%, preferably at least about 95%, more preferably at least about 96 to 98% or more, and in particular embodiments, as high at about 99% or more of the nucleotides, including, e.g., segments encoding structural domains such as the segments described below. Alternatively, substantial identity will exist when the segments will hybridize under selective hybridization conditions, to a strand or its complement, typically using a sequence derived from Table 1. As disclosed herein, typically, selective hybridization will occur when there is at least about 55% homology over a stretch of at least about 14 nucleotides, more typically at least about 65%, preferably at least about 75%, and more preferably at least about 90%.. See, Kanehisa (1984) Nucl. Acids Res. 12:203-213. The length of homology comparison, as described, may be over longer stretches, and in certain embodiments will be over a stretch of at least about 17 nucleotides, generally at least about 20 nucleotides, ordinarily at least about 24 nucleotides, usually at least about 28 nucleotides, typically at least about 32 nucleotides, more typically at least about 40 nucleotides, typically at least about 50 nucleotides, and more typically at least about 75 to 100 or more nucleotides. This includes, e.g., 125, 150, 175, 200, 225, 246, 273, and other lengths.

Stringent conditions, in referring to homology in the hybridization context, will be stringent combined conditions of salt, temperature, organic solvents, and other parameters typically controlled in hybridization reactions. Stringent temperature conditions will usually include temperatures in excess of about 30° C, more usually in excess of about 37° C, typically in excess of about 45° C, more typically in excess of' about 55° C, preferably in excess of about 65° C, and more preferably in excess of about 70° C. Stringent salt conditions will ordinarily be less than about 500 mM, usually less than about 400 mM, more usually less than about 300 mM, typically less than about 200 mM, preferably less than about 100 mM; and more preferably leas than about 80 mM, even down to less than about 20 mM. However, the combination of parameters is much more important than the measure of any single parameter. See, e.g., Wetmur and Davidson (1968) J. Mol. Biol. 31:349-370.

The isolated DNA can be readily modified by nucleotide substitutions, nucleotide deletions, nucleotide insertions, and inversions of nucleotide stretches. These modifications result in novel DNA sequences which encode this protein or its derivatives. These modified sequence can be used to produce mutant proteins (muteins) or to enhance the expression of variant species. Enhanced expression may involve gene amplification, increased transcription, increased translation, and other mechanisms. Such mutant DCRS2-like derivatives include predetermined or site-specific mutations of the protein or its fragments, including silent mutations using genetic code degeneracy. "Mutant DCRS2" as used herein encompasses a polypeptide otherwise falling within the homology definition of the DCRS2 as set forth above, but having an amino acid sequence which differs from that of other cytokine receptor-like proteins as found in nature, whether by way of deletion, substitution, or insertion. In particular, "site specific mutant DCRS2" encompasses a protein having substantial sequence identity with a protein of Table 1, and typically shares most of the biological activities or effects of the forms disclosed herein.

Although site specific mutation sites are predetermined, mutants need not be site specific. Mammalian DCRS2 mutagenesis can be achieved by making amino acid insertions or deletions in the gene, coupled with expression. Substitutions, deletions, insertions, or many combinations may be generated to arrive at a final construct. Insertions include amino- or carboxy-terminal fusions. Random mutagenesis can be conducted at a target codon and the expressed mammalian DCRS2 mutants can then be screened for the desired activity, providing some aspect of a structure-activity relationship. Methods for making substitution mutations at predetermined sites in DNA having a known sequence are well known in the art, e.g., by M13 primer mutagenesis. See also Sambrook, et al. (1989) and Ausubel, et al. (1987 and periodic Supplements).

The mutations in the DNA normally should not place coding sequences out of reading frames and preferably will not create complementary regions that could hybridize to produce secondary mRNA structure such as loops or hairpins.

The phosphoramidite method described by Beaucage and Carruthers (1981) Tetra. Letts. 22:1859-1862, will produce suitable synthetic DNA fragments. A double stranded fragment will often be obtained either by synthesizing the complementary strand and annealing the strand together under appropriate conditions or by adding the complementary strand using DNA polymerase with an appropriate primer sequence.

Polymerase chain reaction (PCR) techniques can often be applied in mutagenesis. Alternatively, mutagenesis primers are commonly used methods for generating defined mutations at predetermined sites. See, e.g., Innis, et al. (eds. 1990) PCR Protocols: A Guide to Methods and Applications Academic Press, San Diego, CA; and Dieffenbach and Dveksler (1995; eds.) PCR Primer: A Laboratory Manual Cold Spring Harbor Press, CSH, NY.

Certain embodiments of the invention are directed to combination compositions comprising the receptor or ligand sequences described insofar as these are encompassed by the appendant claims. In other embodiments, functional portions of the sequences may be joined to encode fusion proteins. In other forms, variants of the described sequences may be substituted.

### IV. Proteins, Peptides

As described above, the present invention encompasses primate DCRS2, e.g., whose sequences are disclosed in Table 1, and described above. Allelic and other variants are also contemplated, including, e.g., fusion proteins combining portions of such sequences with others, including, e.g., epitope tags and functional domains, insofar as these are encompassed by the appendant claims

The present invention also provides recombinant proteins, e.g., heterologous fusion proteins using segments from these primate proteins. A heterologous fusion protein is a fusion of proteins or segments which are naturally not normally fused in the same manner. Thus, the fusion product of a DCRS2 with another cytokine receptor is a continuous protein molecule having sequences fused in a typical peptide linkage, typically made as a single translation product and exhibiting properties, e.g., sequence or antigenicity, derived from each source peptide. A similar concept applies to heterologous nucleic acid sequences. Combinations of various designated proteins into complexes are also provided.

In addition, new constructs may be made from combining similar functional or structural domains from other related proteins, e.g., cytokine receptors or Toll-like receptors, including species variants. For example, ligand-binding or other segments may be "swapped" between different new fusion polypeptides or fragments. See, e.g., Cunningham, et al. (1989) Science 243:1330-1336; and O'Dowd, et al. (1988) J. Biol. Chem. 263:15985-15992. Thus, new chimeric polypeptides exhibiting new combinations of specificities will result from the functional linkage of receptor-binding specificities. For example, the ligand binding domains from other related receptor molecules may be added or substituted for other domains of this or related proteins. The resulting protein will often have hybrid function and properties. For example, a fusion protein may include a targeting domain which may serve to provide sequestering of the fusion protein to a particular subcellular organelle.

Candidate fusion partners and sequences can be selected from various sequence data bases, e.g., GenBank, c/o IntelliGenetics, Mountain View, CA; and BCG, University of Wisconsin Biotechnology Computing Group, Madison, WI, which are each incorporated herein by reference. In particular, combinations of polypeptide sequences provided in Tables 1 and 3 are particularly preferred. Variant forms of the proteins may be substituted in the described combinations.

The present invention particularly provides muteins which bind cytokine-like ligands, and/or which are affected in signal transduction. Structural alignment of human DCRS2 with other members of the cytokine receptor family show conserved features/residues. See Table 3. Alignment of the human DCRS2 sequence with other members of the cytokine receptor family indicates various structural and functionally shared features. See also, Bazan, et al. (1996) Nature 379:591; Lodi, et al. (1994) Science 263:1762-1766; Sayle and Milner-White (1995) TIBS 20:374-376; and Gronenberg, et al. (1991) Protein Engineering 4:263-269.

Substitutions with either mouse sequences or human sequences are particularly preferred. Conversely, conservative substitutions away from the ligand binding interaction regions will probably preserve most signaling activities; and conservative substitutions away from the intracellular domains will probably preserve most ligand binding properties.

"Derivatives" of the primate DCRS2 include amino acid sequence mutants, glycosylation variants, metabolic derivatives and covalent or aggregative conjugates with other chemical moieties. Covalent derivatives can be prepared by linkage of functionalities to groups which are found in the DCRS2 amino acid side chains or at the N- or C- termini, e.g., by means which are well known in the art. These derivatives can include, without limitation, aliphatic esters or amides of the carboxyl terminus, or of residues containing carboxyl side chains, O-acyl derivatives of hydroxyl group-containing residues, and N-acyl derivatives of the amino terminal amino acid or amino-group containing residues, e.g., lysine or arginine. Acyl groups are selected from the group of alkyl-moieties, including C3 to C18 normal alkyl, thereby forming alkanoyl aroyl species.

In particular, glycosylation alterations are included, e.g., made by modifying the glycosylation patterns of a polypeptide during its synthesis and processing, or in further processing steps. Particularly preferred means for accomplishing this are by exposing the polypeptide to glycosylating enzymes derived from cells which normally provide such processing, e.g., mammalian glycosylation enzymes. Deglycosylation enzymes are also contemplated. Also embraced are versions of the same primary amino acid sequence which have other minor modifications, including phosphorylated amino acid residues, e.g., phosphotyrosine, phosphoserine, or phosphothreonine.

A major group of derivatives are covalent conjugates of the receptors or fragments thereof with other proteins of polypeptides. These derivatives can be synthesized in recombinant culture such as N- or C-terminal fusions or by the use of agents known in the art for their usefulness in cross-linking proteins through reactive side groups. Preferred derivatization sites with cross-linking agents are at free amino groups, carbohydrate moieties, and cysteine residues.

Fusion polypeptides between the receptors and other homologous or heterologous proteins are also provided. Homologous polypeptides may be fusions between different receptors, resulting in, for instance, a hybrid protein exhibiting binding specificity for multiple different cytokine ligands, or a receptor which may have broadened or weakened specificity of substrate effect. Likewise, heterologous fusions may be constructed which would exhibit a combination of properties or activities of the derivative proteins. Typical examples are fusions of a reporter polypeptide, e.g., luciferase, with a segment or domain of a receptor, e.g., a ligand-binding segment, so that the presence or location of a desired ligand may be easily determined. See, e.g., Dull, et al., U.S. Patent No. 4,859,609. Other gene fusion partners include glutathione-S-transferase (GST), bacterial ß-galactosidase, trpE, Protein A, ß-lactamase, alpha amylase, alcohol dehydrogenase, and yeast alpha mating factor. See, e.g., Godowski, et al. (1988) Science 241:812-816. Labeled proteins will often be substituted in the described combinations of proteins.

The phosphoramidite method described by Beaucage and Carruthers (1981) Tetra. Letts. 22:1859-1862, will produce suitable synthetic DNA fragments. A double stranded fragment will often be obtained either by synthesizing the complementary strand and annealing the strand together under appropriate conditions or by adding the complementary strand using DNA polymerase with an appropriate primer sequence.

Such polypeptides may also have amino acid residues which have been chemically modified by phosphorylation, sulfonation, biotinylation, or the addition or removal of other moieties, particularly those which have molecular shapes similar to phosphate groups. In some embodiments, the modifications will be useful labeling reagents, or serve as purification targets, e.g., affinity ligands.

Fusion proteins will typically be made by either recombinant nucleic acid methods or by synthetic polypeptide methods. Techniques for nucleic acid manipulation and expression are described generally, for example, in Sambrook, et al. (1989) Molecular Cloning: A Laboratory Manual. (2d ed.), Vols. 1-3, Cold Spring Harbor Laboratory, and Ausubel, et al. (eds. 1987 and periodic supplements) Current Protocols in Molecular Biology, Greene/Wiley, New York, which are each incorporated herein by reference. Techniques for synthesis of polypeptides are described, for example, in Merrifield (1963) J. Amer. Chem. Soc. 85:2149-2156; Merrifield (1986) Science 232: 341-347; and Atherton, et al. (1989) Solid Phase Peptide Synthesis: A Practical Approach, IRL Press, Oxford. See also Dawson, et al. (1994) Science 266:776-779 for methods to make larger polypeptides.

This invention also contemplates the use of derivatives of a DCRS2 other than variations in amino acid sequence or glycosylation. Such derivatives may involve covalent or aggregative association with chemical moieties. These derivatives generally fall into three classes: (1) salts, (2) side chain and terminal residue covalent modifications, and (3) adsorption complexes, for example with cell membranes. Such covalent or aggregative derivatives are useful as immunogens, as reagents in immunoassays, or in purification methods such as for affinity purification of a receptor or other binding molecule, e.g., an antibody. For example, a cytokine ligand can be immobilized by covalent bonding to a solid support such as cyanogen bromide-activated Sepharose, by methods which are well known in the art, or adsorbed onto polyolefin surfaces, with or without glutaraldehyde cross-linking, for use in the assay or purification of a cytokine receptor, antibodies, or other similar molecules. The ligand can also be labeled with a detectable group, for example radioiodinated by the chloramine T procedure, covalently bound to rare earth chelates, or conjugated to another fluorescent moiety for use in diagnostic assays.

A combination, e.g., including a DCRS2, of this invention can be used as an immunogen for the production of antisera or antibodies specific, e.g., capable of distinguishing between other cytokine receptor family members, for the combinations described. The complexes can be used to screen monoclonal antibodies or antigen-binding fragments prepared by immunization with various forms of impure preparations containing the protein. In particular, the term "antibodies" also encompasses antigen binding fragments of natural antibodies, e.g., Fab, Fab2, Fv, etc. The purified DCRS2 can also be used as a reagent to detect antibodies generated in response to the presence of elevated levels of expression, or immunological disorders which lead to antibody production to the endogenous receptor. Additionally, DCRS2 fragments may also serve as immunogens to produce the antibodies of the present invention, as described immediately below insofar as these are encompassed by the appendant claims. For example, this invention contemplates antibodies having binding affinity to or being raised against the amino acid sequences shown in Table 1, fragments thereof, or various homologous peptides. In particular, this invention contemplates antibodies having binding affinity to, or having been raised against, specific fragments which are predicted to be, or actually are, exposed at the exterior protein surface of the native DCRS2. Complexes of combinations of proteins will also be useful, and antibody preparations thereto can be made.

The blocking of physiological response to the receptor ligands may result from the inhibition of binding of the ligand to the receptor, likely through competitive inhibition. Thus, in vitro assays of the present invention will often use antibodies or antigen binding segments of these antibodies, or fragments attached to solid phase substrates. These assays will also allow for the diagnostic determination of the effects of either ligand binding region mutations and modifications, or other mutations and modifications, e.g., which affect signaling or enzymatic function.

This invention also contemplates the use of competitive drug screening assays, e.g., where neutralizing antibodies to the receptor complexes or fragments compete with a test compound for binding to a ligand or other antibody. In this manner, the neutralizing antibodies or fragments can be used to detect the presence of a polypeptide which shares one or more binding sites to a receptor and can also be used to occupy binding sites on a receptor that might otherwise bind a ligand.

### V. Making Nucleic Acids and Protein

DNA which encodes the protein or fragments thereof can be obtained by chemical synthesis, screening cDNA libraries, or by screening genomic libraries prepared from a wide variety of cell lines or tissue samples. Natural sequences can be isolated using standard methods and the sequences provided herein, e.g., in Table 1. Other species counterparts can be identified by hybridization techniques, or by various PCR techniques, combined with or by searching in sequence databases, e.g., GenBank.

This DNA can be expressed in a wide variety of host cells for the synthesis of a full-length receptor or fragments which can in turn, for example, be used to generate polyclonal or monoclonal antibodies; for binding studies; for construction and expression of modified ligand binding or kinase/phosphatase domains; and for structure/function studies. Variants or fragments can be expressed in host cells that are transformed or transfected with appropriate expression vectors. These molecules can be substantially free of protein or cellular contaminants, other than those derived from the recombinant host, and therefore are particularly useful in pharmaceutical compositions when combined with a pharmaceutically acceptable carrier and/or diluent. The protein, or portions thereof, may be expressed as fusions with other proteins. Combinations of the described proteins, or nucleic acids encoding them, are particularly interesting.

Expression vectors are typically self-replicating DNA or RNA constructs containing the desired receptor gene or its fragments, usually operably linked to suitable genetic control elements that are recognized in a suitable host cell. These control elements are capable of effecting expression within a suitable host. The multiple genes may be coordinately expressed, and may be on a polycistronic message. The specific type of control elements necessary to effect expression will depend upon the eventual host cell used. Generally, the genetic control elements can include a prokaryotic promoter system or a eukaryotic promoter expression control system, and typically include a transcriptional promoter, an optional operator to control the onset of transcription, transcription enhancers to elevate the level of mRNA expression, a sequence that encodes a suitable ribosome binding site, and sequences that terminate transcription and translation. Expression vectors also usually contain an origin of replication that allows the vector to replicate independently of the host cell.

The vectors of this invention include those which contain DNA which encodes a a combination of proteins, as described, or a biologically active equivalent polypeptide insofar as these are encompassed by the appendant claims. The DNA can be under the control of a viral promoter and can encode a selection marker. This invention further contemplates use of such expression vectors which are capable of expressing eukaryotic cDNAs coding for such proteins in a prokaryote or eukaryotic host cell, where the vector is compatible with the host cell and where the eukaryotic cDNAs, are inserted into the vector such that growth of the host cell containing the vector expresses the cDNAs in question. Usually, expression vectors are designed for stable replication in their host cells or for amplification to greatly increase the total number of copies of the desirable gene per cell. It is not always necessary to require that an expression vector replicate in a host cell, e.g., it is possible to effect transient expression of the protein or its fragments in various hosts using vectors that do not contain a replication origin that is recognized by the host cell. It is also possible to use vectors that cause integration of the protein encoding portions into the host cell DNA by recombination.

Vectors, as used herein, comprise plasmids, viruses, bacteriophage, integratable DNA fragments, and other vehicles which enable the integration of DNA fragments into the genome of the host cell. Expression vectors are specialized vectors which contain genetic control elements that effect expression of operably linked genes. Plasmids are the most commonly used form of vector but all other forms of vectors which serve an equivalent function and which are, or become, known in the art are suitable for use herein. See, e.g., Pouwels, et al. (1985 and Supplements) Cloning Vectors: A Laboratory Manual, Elsevier, N.Y., and Rodriguez, et al. (eds. 1988) Vectors: A Survey of Molecular Cloning Vectors and Their Uses, Buttersworth, Boston.

Transformed cells are cells, preferably mammalian, that have been transformed or transfected with vectors constructed using recombinant DNA techniques. Transformed host cells usually express the desired proteins, but for purposes of cloning, amplifying, and manipulating its DNA, do not need to express the subject proteins. This invention further contemplates culturing transformed cells in a nutrient medium, thus permitting the proteins to accumulate. The proteins can be recovered, either from the culture or, in certain instances, from the culture medium.

For purposes of this invention, nucleic sequences are operably linked when they are functionally related to each other. For example, DNA for a presequence or secretory leader is operably linked to a polypeptide if it is expressed as a preprotein or participates in directing the polypeptide to the cell membrane or in secretion of the polypeptide. A promoter is operably linked to a coding sequence if it controls the transcription of the polypeptide; a ribosome binding site is operably linked to a coding sequence if it is positioned to permit translation. Usually, operably linked means contiguous and in reading frame, however, certain genetic elements such as repressor genes are not contiguously linked but still bind to operator sequences that in turn control expression.

Suitable host cells include prokaryotes, lower eukaryotes, and higher eukaryotic cells. Prokaryotes include both gram negative and gram positive organisms, e.g., E. coli and B. subtilis. Lower eukaryotes include yeasts, e.g., S. cerevisiae and Pichia, and species of the genus Dictyostelium. Higher eukaryotic cells include established tissue culture cell lines from animal cells, both of non-mammalian origin, e.g., insect cells, and birds, and of mammalian origin, e.g., human, primates, and rodents.

Prokaryotic host-vector systems include a wide variety of vectors for many different species. As used herein, E. coli and its vectors will be used generically to include equivalent vectors used in other prokaryotes. A representative vector for amplifying DNA is pBR322 or many of its derivatives. Vectors that can be used to express the receptor or its fragments include, but are not limited to, such vectors as those containing the lac promoter (pUC-series); trp promoter (pBR322-trp); Ipp promoter (the pIN-series); lambda-pP or pR promoters (pOTS); or hybrid promoters such as ptac (pDR540). See Brosius, et al. (1988) "Expression Vectors Employing Lambda-, trip-, lac-, and Ipp-derived Promoters", in Vectors: A Survey of Molecular Cloning Vectors and Their Uses, (eds. Rodriguez and Denhardt), Buttersworth, Boston, Chapter 10, pp. 205-236.

Lower eukaryotes, e.g., yeasts and Dictyostelium, may be transformed with DCRS2 sequence containing vectors. For purposes of this invention, the most common lower eukaryotic host is the baker's yeast, Saccharomyces cerevisiae. It will be used to generically represent lower eukaryotes although a number of other strains and species are also available. Yeast vectors typically consist of a replication origin (unless of the integrating type), a selection gene, a promoter, DNA encoding the receptor or its fragments, and sequences for translation termination, polyadenylation, and transcription termination. Suitable expression vectors for yeast include such constitutive promoters as 3-phosphoglycerate kinase and various other glycolytic enzyme gene promoters or such inducible promoters as the alcohol dehydrogenase 2 promoter or metallothionine promoter. Suitable vectors include derivatives of the following types: self-replicating low copy number (such as the YRp-series), self-replicating high copy number (such as the YEp-series); integrating types (such as the YIp-series), or mini-chromosomes (such as the YCp-series).

Higher eukaryotic tissue culture cells are normally the preferred host cells for expression of the functionally active interleukin or receptor proteins. In principle, many higher eukaryotic tissue culture cell lines are workable, e.g., insect baculovirus expression systems, whether from an invertebrate or vertebrate source. However, mammalian cells are preferred. Transformation or transfection and propagation of such cells has become a routine procedure. Examples of useful cell lines include HeLa cells, Chinese hamster ovary (CHO) cell lines, baby rat kidney (BRK) cell lines, insect cell lines, bird cell lines, and monkey (COS) cell lines. Expression vectors for such cell lines usually include an origin of replication, a promoter, a translation initiation site, RNA splice sites (if genomic DNA is used), a polyadenylation site, and a transcription termination site. These vectors also usually contain a selection gene or amplification gene. Suitable expression vectors may be plasmids, viruses, or retroviruses carrying promoters derived, e.g., from such sources as from adenovirus, SV40, parvoviruses, vaccinia virus, or cytomegalovirus. Representative examples of suitable expression vectors include pCDNA1; pCD, see Okayama, et al. (1985) Mol. Cell Biol. 5:1136-1142; pMClneo PolyA, see Thomas, et al. (1987) Cell 51:503-512; and a baculovirus vector such as pAC 373 or pAC 610.

For secreted proteins and some membrane proteins, an open reading frame usually encodes a polypeptide that consists of a mature or secreted product covalently linked at its N-terminus to a signal peptide. The signal peptide is cleaved prior to secretion of the mature, or active, polypeptide. The cleavage site can be predicted with a high degree of accuracy from empirical rules, e.g., von-Heijne (1986) Nucleic Acids Research 14:4683-4690 and Nielsen, et al. (1997) Protein Eng. 10:1-12, and the precise amino acid composition of the signal peptide often does not appear to be critical to its function, e.g., Randall, et al. (1989) Science 243:1156-1159; Kaiser et al. (1987) Science 235:312-317. The mature proteins of the invention can be readily determined using standard methods.

It will often be desired to express these polypeptides in a system which provides a specific or defined glycosylation pattern. In this case, the usual pattern will be that provided naturally by the expression system. However, the pattern will be modifiable by exposing the polypeptide, e.g., an unglycosylated form, to appropriate glycosylating proteins introduced into a heterologous expression system. For example, the receptor gene may be co-transformed with one or more genes encoding mammalian or other glycosylating enzymes. Using this approach, certain mammalian glycosylation patterns will be achievable in prokaryote or other cells. Expression in prokaryote cells will typically lead to unglycosylated forms of protein.

The source of DCRS2 can be a eukaryotic or prokaryotic host cell expressing recombinant DCRS2, such as is described above. The source can also be a cell line, but other mammalian cell lines are also contemplate by this invention, with the preferred cell line being from the human species.

Now that the sequences are known, the primate DCRS2, fragments, or derivatives thereof can be prepared by conventional processes for synthesizing peptides. These include processes such as are described in Stewart and Young (1984) Solid Phase Peptide Syntheses, Pierce Chemical Co., Rockford, IL; Bodanszky and Bodanszky (1984) The Practice of Peptide Synthesis, Springer-Verlag, New York; and Bodanszky (1984) The Principles of Peptide Synthesis, Springer-Verlag, New York. For example, an azide process, an acid chloride process, an acid anhydride process, a mixed anhydride process, an active ester process (for example, p-nitrophenyl ester, N-hydroxysuccinimide ester, or cyanomethyl ester), a carbodiimidazole process, an oxidative-reductive process, or a dicyclohexylcarbodiimide (DCCD)/additive process can be used. Solid phase and solution phase syntheses are both applicable to the foregoing processes. Similar techniques can be used with partial DCRS2 sequences.

The DCRS2 proteins, fragments, or derivatives are suitably prepared in accordance with the above processes as typically employed in peptide synthesis, generally either by a so-called stepwise process which comprises condensing an amino acid to the terminal amino acid, one by one in sequence, or by coupling peptide fragments to the terminal amino acid. Amino groups that are not being used in the coupling reaction typically must be protected to prevent coupling at an incorrect location.

If a solid phase synthesis is adopted, the C-terminal amino acid is bound to an insoluble carrier or support through its carboxyl group. The insoluble carrier is not particularly limited as long as it has a binding capability to a reactive carboxyl group. Examples of such insoluble carriers include halomethyl resins, such as chloromethyl resin or bromomethyl resin, hydroxymethyl resins, phenol resins, tert-alkyloxycarbonylhydrazidated resins, and the like.

An amino group-protected amino acid is bound in sequence through condensation of its activated carboxyl group and the reactive amino group of the previously formed peptide or chain, to synthesize the peptide step by step. After synthesizing the complete sequence, the peptide is split off from the insoluble carrier to produce the peptide. This solid-phase approach is generally described by Merrifield, et al. (1963) in J. Am. Chem. Soc. 85:2149-2156.

The prepared protein and fragments thereof can be isolated and purified from the reaction mixture by means of peptide separation, for example, by extraction, precipitation, electrophoresis, various forms of chromatography, and the like. The receptors of this invention can be obtained in varying degrees of purity depending upon desired uses. Purification can be accomplished by use of the protein purification techniques disclosed herein, see below, or by the use of the antibodies herein described in methods of immunoabsorbant affinity chromatography. This immunoabsorbant affinity chromatography is carried out by first linking the antibodies to a solid support and then contacting the linked antibodies with solubilized lysates of appropriate cells, lysates of other cells expressing the receptor, or lysates or supernatants of cells producing the protein as a result of DNA techniques, see below.

Generally, the purified protein will be at least about 40% pure, ordinarily at least about 50% pure, usually at least about 60% pure, typically at least about 70% pure, more typically at least about 80% pure, preferable at least about 90% pure and more preferably at least about 95% pure, and in particular embodiments, 97%-99% or more. Purity will usually be on a weight basis, but can also be on a molar basis. Different assays will be applied as appropriate. Individual proteins may be purified and thereafter combined.

### VI. Antibodies

Antibodies can be raised to the various mammalian, e.g., primate DCRS2 proteins and fragments thereof, both in naturally occurring native forms and in their recombinant forms, the difference being that antibodies to the active receptor are more likely to recognize epitopes which are only present in the native conformations. Denatured antigen detection can also be useful in, e.g., Western analysis. Anti-idiotypic antibodies are also contemplated, which would be useful as agonists or antagonists of a natural receptor or an antibody.

Antibodies, including binding fragments and single chain versions, against predetermined fragments of the protein can be raised by immunization of animals with conjugates of the fragments with immunogenic proteins. Monoclonal antibodies are prepared from cells secreting the desired antibody. These antibodies can be screened for binding to normal or defective protein, or screened for agonistic or antagonistic activity. These monoclonal antibodies will usually bind with at least a K_{D} of about 1 mM, more usually at least about 300 µM, typically at least about 100µM, more typically at least about 30 µM, preferably at least about 10 µM, and more preferably at least about 3 µM or better.

The antibodies, including antigen binding fragments, of this invention can have significant diagnostic or therapeutic value. They can be potent antagonists that bind to the receptor and inhibit binding to ligand or inhibit the ability of the receptor to elicit a biological response, e.g., act on its substrate. They also can be useful as non-neutralizing antibodies and can be coupled to toxins or radionuclides to bind producing cells, or cells localized to the source of the interleukin. Further, these antibodies can be conjugated to drugs or other therapeutic agents, either directly or indirectly by means of a linker.

The antibodies of this invention can also be useful in diagnostic applications. As capture or non-neutralizing antibodies, they might bind to the receptor without inhibiting ligand or substrate binding. As neutralizing antibodies, they can be useful in competitive binding assays. They will also be useful in detecting or quantifying ligand. They may be used as reagents for Western blot analysis, or for immunoprecipitation or immunopurification of the respective protein. Likewise, nucleic acids and proteins may be immobilized to solid substrates for affinity purification or detection methods. The substrates may be, e.g., solid resin beads or sheets of plastic.

Protein fragments may be joined to other materials, particularly polypeptides, as fused or covalently joined polypeptides to be used as immunogens. Mammalian cytokine receptors and fragments may be fused or covalently linked to a variety of immunogens, such as keyhole limpet hemocyanin, bovine serum albumin, tetanus toxoid, etc. See Microbiology, Hoeber Medical Division, Harper and Row, 1969; Landsteiner (1962) Specificity of Serological Reactions, Dover Publications, New York; and Williams, et al. (1967) Methods in Immunology and Immunochemistry, Vol. 1, Academic Press, New York, for descriptions of methods of preparing polyclonal antisera. A typical method involves hyperimmunization of an animal with an antigen. The blood of the animal is then collected shortly after the repeated immunizations and the gamma globulin is isolated.

In some instances, it is desirable to prepare monoclonal antibodies from various mammalian hosts, such as mice, rodents, primates, humans, etc. Description of techniques for preparing such monoclonal antibodies may be found in, e.g., Stites, et al. (eds.) Basic and Clinical immunology (4th ed.), Lange Medical Publications, Los Altos, CA, and references cited therein; Harlow and Lane (1988) Antibodies: A Laboratory Manual, CSH Press; Goding (1986) Monoclonal Antibodies: Principles and Practice (2d ed.) Academic Press, New York; and particularly in Kohler and Milstein (1975) in Nature 256: 495-497, which discusses one method of generating monoclonal antibodies. Summarized briefly, this method involves injecting an animal with an immunogen. The animal is then sacrificed and cells taken from its spleen, which are then fused with myeloma cells. The result is a hybrid cell or "hybridoma" that is capable of reproducing in vitro. The population of hybridomas is then screened to isolate individual clones, each of which secrete a single antibody species to the immunogen. In this manner, the individual antibody species obtained are the products of immortalized and cloned single B cells from the immune animal generated in response to a specific site recognized on the immunogenic substance.

Other suitable techniques involve in vitro exposure of lymphocytes to the antigenic polypeptides or alternatively to selection of libraries of antibodies in phage or similar vectors. See, Huse, et al. (1989) "Generation of a Large Combinatorial Library of the Immunoglobulin Repertoire in Phage Lambda," Science 246:1275-1281; and ward, et al. (1989) Nature 341:544-546,

The polypeptides and antibodies of the present invention may be used with or without modification, including chimeric or humanized antibodies. Frequently, the polypeptides and antibodies will be labeled by joining, either covalently or non-covalently, a substance which provides for a detectable signal. A wide variety of labels and conjugation techniques are known and are reported extensively in both the scientific and patent literature. Suitable labels include radionuclides, enzymes, substrates, cofactors, inhibitors, fluorescent moieties, chemiluminescent moieties, magnetic particles, and the like. Patents, teaching the use of such labels include U.S. Patent Nos. 3,817,837; 3,850,752; 3,939,350; 3,996,345; 4,277,437; 4,275,149; and 4,366,241. Also, recombinant or chimeric immunoglobulins may be produced, see Cabilly, U.S. Patent No. 4,816,567; or made in transgenic mice, see Mendez, et al. (1997) Nature Genetics 15:146-156.

The antibodies of this invention can also be used for affinity chromatography in isolating the DCRS2 proteins or peptides. Columns can be prepared where the antibodies are linked to a solid support, e.g., particles, such as agarose, Sephadex, or the like, where a cell lysate may be passed through the column, the column washed, followed by increasing concentrations of a mild denaturant, whereby the purified protein will be released. Alternatively, the protein may be used to purify antibody. Appropriate cross absorptions or depletions may be applied.

The antibodies may also be used to screen expression libraries for particular expression products. Usually the antibodies used in such a procedure will be labeled with a moiety allowing easy detection of presence of antigen by antibody binding.

Antibodies raised against a cytokine receptor will also be used to raise anti-idiotypic antibodies. These will be useful in detecting or diagnosing various immunological conditions related to expression of the protein or cells which express the protein. They also will be useful as agonists or antagonists of the ligand, which may be competitive inhibitors or substitutes for naturally occurring ligands.

A cytokine receptor protein that specifically binds to or that is specifically immunoreactive with an antibody generated against a defined immunogen, such as an immunogen consisting of the amino acid sequence of SEQ ID NO: 2, is typically determined in an immunoassay. The immunoassay typically uses a polyclonal antiserum which was raised, e.g., to a protein of SEQ ID NO: 2. This antiserum is selected to have low crossreactivity against other cytokine receptor family members, e.g., IL-11 receptor subunit alpha, IL-6 receptor subunit alpha, or p40, preferably from the same species, and any such crossreactivity is removed by immunoabsorption prior to use in the immunoassay.

In order to produce antisera for use in an immunoassay, the protein, e.g., of SEQ ID NO: 2, is isolated as described herein. For example, recombinant protein may be produced in a mammalian cell line. An appropriate host, e.g., an inbred strain of mice such as Balb/c, is immunized with the selected protein, typically using a standard adjuvant, such as Freund's adjuvant, and a standard mouse immunization protocol (see Harlow and Lane, supra). Alternatively, a synthetic peptide derived from the sequences disclosed herein and conjugated to a carrier protein can be used an immunogen. Polyclonal sera are collected and titered against the immunogen protein in an immunoassay, e.g., a solid phase immunoassay with the immunogen immobilized on a solid support. Polyclonal antisera with a titer of 10⁴ or greater are selected and tested for their cross reactivity against other cytokine receptor family members, e.g., IL-11 receptor subunit alpha and/or p40, using a competitive binding immunoassay such as the one described in Harlow and Lane, supra, at pages 570-573. Preferably at least two cytokine receptor family members are used in this determination. These cytokine receptor family members can be produced as recombinant proteins and isolated using standard molecular biology and protein chemistry techniques as described herein.

Immunoassays in the competitive binding format can be used for the crossreactivity determinations. For example, the protein of SEQ ID NO: 2 can be immobilized to a solid support. Proteins added to the assay compete with the binding of the antisera to the immobilized antigen. The ability of the above proteins to compete with the binding of the antisera to the immobilized protein is compared to the proteins, e.g., of IL-11 receptor subunit alpha or p40. The percent crossreactivity for the above proteins is calculated, using standard calculations. Those antisera with less than 10% crossreactivity with each of the proteins listed above are selected and pooled. The cross-reacting antibodies are then removed from the pooled antisera by immunoabsorption with the above-listed proteins.

The immunoabsorbed and pooled antisera are then used in a competitive binding immunoassay as described above to compare a second protein to the immunogen protein (e.g., the DCRS2 like protein of SEQ ID NO: 2). In order to make this comparison, the two proteins are each assayed at a wide range of concentrations and the amount of each protein required to inhibit 50% of the binding of the antisera to the immobilized protein is determined. If the amount of the second protein required is less than twice the amount of the protein of the selected protein or proteins that is required, then the second protein is said to specifically bind to an antibody generated to the immunogen.

It is understood that these cytokine receptor proteins are members of a family of homologous proteins that comprise at least 6 so far identified genes. For a particular gene product, such as the DCRS2, the term refers not only to the amino acid sequences disclosed herein, but also to other proteins that are allelic, non-allelic, or species variants. It is also understood that the terms include nonnatural mutations introduced by deliberate mutation using conventional recombinant technology such as single site mutation, or by excising short sections of DNA encoding the respective proteins, or by substituting new amino acids, or adding new amino acids. Such minor alterations typically will' substantially maintain the immunoidentity of the original molecule and/or its biological activity. Thus, these alterations include proteins that are specifically immunoreactive with a designated naturally occurring DCRS2 protein. The biological properties of the altered proteins can be determined by expressing the protein in an appropriate cell line and measuring the appropriate effect, e.g., upon transfected lymphocytes. Particular protein modifications considered minor would include conservative substitution of amino acids with similar chemical properties, as described above for the cytokine receptor family as a whole. By aligning a protein optimally with the protein of the cytokine receptors and by using the conventional immunoassays described herein to determine immunoidentity, one can determine the protein compositions of the invention.

### VII. Kits and quantitation

Both naturally occurring and recombinant forms of the cytokine receptor like molecules of this invention are particularly useful in kits and assay methods. For example, these methods would also be applied to screening for binding activity, e.g., ligands for these proteins. Several methods of automating assays have been developed in recent years so as to permit screening of tens of thousands of compounds per year. See, e.g., a BIOMEK automated workstation, Beckman Instruments, Palo Alto, California, and Fodor, et al. (1991) Science 251:767-773. The latter describes means for testing binding by a plurality of defined polymers synthesized on a solid substrate. The development of suitable assays to screen for a ligand or agonist/antagonist homologous proteins can be greatly facilitated by the availability of large amounts of purified, soluble cytokine receptors in an active state such as is provided by this invention.

Purified DCRS2 can be coated directly onto plates for use in the aforementioned ligand screening techniques. However, non-neutralizing antibodies to these proteins can be used as capture antibodies to immobilize the respective receptor on the solid phase, useful, e.g., in diagnostic uses.

This invention also contemplates use of DCRS2, fragments thereof, peptides, and their fusion products in a variety of diagnostic kits and methods for detecting the presence of the protein or its ligand. Alternatively, or additionally, antibodies against the molecules may be incorporated into the kits and methods. Typically the kit will have a compartment containing either a DCRS2 peptide or gene segment or a reagent which recognizes one or the other. Typically, recognition reagents, in the case of peptide, would be a receptor or antibody, or in the case of a gene segment, would usually be a hybridization probe.

A preferred kit for determining the concentration of DCRS2 in a sample would typically comprise a labeled compound, e.g., ligand or antibody, having known binding affinity for DCRS2, a source of DCRS2 (naturally occurring or recombinant) as a positive control, and a means for separating the bound from free labeled compound, for example a solid phase for immobilizing the DCRS2 in the test sample. Compartments containing reagents, and instructions, will normally be provided. Appropriate nucleic acid or protein containing kits are also provided.

Antibodies, including antigen binding fragments, specific for mammalian DCRS2 or a peptide fragment, or receptor fragments are useful in diagnostic applications to detect the presence of elevated levels of ligand and/or its fragments. Diagnostic assays may be homogeneous (without a separation step between free reagent and antibody-antigen complex) or heterogeneous (with a separation step). Various commercial assays exist, such as radioimmunoassay (RIA), enzyme-linked immunosorbent assay (ELISA), enzyme immunoassay (EIA), enzyme-multiplied immunoassay technique (EMIT), substrate-labeled fluorescent immunoassay (SLFIA) and the like. For example, unlabeled antibodies can be employed by using a second antibody which is labeled and which recognizes the antibody to a cytokine receptor or to a particular fragment thereof. These assays have also been extensively discussed in the literature. See, e.g., Harlow and Lane (1988) Antibodies: A Laboratory Manual, CSH., and Coligan (ed. 1991 and periodic supplements) Current Protocols In Immunology Greene/Wiley, New York.

Anti-idiotypic antibodies may have similar use to serve as agonists or antagonists of cytokine receptors. These should be useful as therapeutic reagents under appropriate circumstances.

Frequently, the reagents for diagnostic assays are supplied in kits, so as to optimize the sensitivity of the assay. For the subject invention, depending upon the nature of the assay, the protocol, and the label, either labeled or unlabeled antibody, or labeled ligand is provided. This is usually in conjunction with other additives, such as buffers, stabilizers, materials necessary for signal production such as substrates for enzymes, and the like. Preferably, the kit will also contain instructions for proper use and disposal of the contents after use. Typically the kit has compartments for each useful reagent, and will contain instructions for proper use and disposal of reagents. Desirably, the reagents are provided as a dry lyophilized powder, where the reagents may be reconstituted in an aqueous medium having appropriate concentrations for performing the assay.

The aforementioned constituents of the diagnostic assays may be used without modification or may be modified in a variety of ways. For example, labeling may be achieved by covalently or non-covalently joining a moiety which directly or indirectly provides a detectable signal. In many of these assays, a test compound, cytokine receptor, or antibodies thereto can be labeled either directly or indirectly. Possibilities for direct labeling include label groups: radiolabels such as ¹²⁵I, enzymes (U.S. Pat. No. 3,645,090) such as peroxidase and alkaline phosphatase, and fluorescent labels (U.S. Pat. No. 3,940,475) capable of monitoring the change in fluorescence intensity, wavelength shift, or fluorescence polarization.

Possibilities for indirect labeling include biotinylation of one constituent followed by binding to avidin coupled to one of the above label groups.

There are also numerous methods of separating the bound from the free ligand, or alternatively the bound from the free test compound. The cytokine receptor can be immobilized on various matrixes followed by washing. Suitable matrices include plastic such as an ELISA plate, filters, and beads. Methods of immobilizing the receptor to a matrix include, without limitation, direct adhesion to plastic, use of a capture antibody, chemical coupling, and biotin-avidin. The last step in this approach involves the precipitation of antibody/antigen complex by any of several methods including those utilizing, e.g., an organic solvent such as polyethylene glycol or a salt such as ammonium sulfate. Other suitable separation techniques include, without limitation, the fluorescein antibody magnetizable particle method described in Rattle, et al. (1984) Clin. Chem. 30(9):1457-1461, and the double antibody magnetic particle separation as described in U.S. Pat. No. 4,659,678.

The methods for linking protein or fragments to various labels have been extensively reported in the literature and do not require detailed discussion here. Many of the techniques involve the use of activated carboxyl groups either through the use of carbodiimide or active esters to form peptide bonds, the formation of thioethers by reaction of a mercapto group with an activated halogen such as chloroacetyl, or an activated olefin such as maleimide, for linkage, or the like. Fusion proteins will also find use in these applications.

Another diagnostic aspect of this invention involves use of oligonucleotide or polynucleotide sequences taken from the sequence of an cytokine receptor. These sequences can be used as probes for detecting levels of the respective cytokine receptor in patients suspected of having an immunological disorder. The preparation of both RNA and DNA nucleotide sequences, the labeling of the sequences, and the preferred size of the sequences has received ample description and discussion in the literature. Normally an oligonucleotide probe should have at least about 14 nucleotides, usually at least about 18 nucleotides, and the polynucleotide probes may be up to several kilobases. Various labels may be employed, most commonly radionuclides, particularly ³²P. However, other techniques may also be employed, such as using biotin modified nucleotides for introduction into a polynucleotide. The biotin then serves as the site for binding to avidin or antibodies, which may be labeled with a wide variety of labels, such as radionuclides, fluorescers, enzymes, or the like. Alternatively, antibodies may be employed which can recognize specific duplexes, including DNA duplexes, RNA duplexes, DNA-RNA hybrid duplexes, or DNA-protein duplexes. The antibodies in turn may be labeled and the assay carried out where the duplex is bound to a surface, so that upon the formation of duplex on the surface, the presence of antibody bound to the duplex can be detected. The use of probes to the novel-anti-sense RNA may be carried out in conventional techniques such as nucleic acid hybridization, plus and minus screening, recombinational probing, hybrid released translation (HRT), and hybrid arrested translation (HART). This also includes amplification techniques such as polymerase chain reaction (PCR).

Diagnostic kits which also test for the qualitative or quantitative presence of other markers are also contemplated. Diagnosis or prognosis may depend on the combination of multiple indications used as markers. Thus, kits may test for combinations of markers. See, e.g., Viallet, et al. (1989) Progress in Growth Factor Res. 1:89-97.

### VIII. Therapeutic Utility

This invention provides reagents with significant therapeutic value. See, e.g., Levitzki (1996) Curr. Opin. Cell Biol. 8:239-244. The cytokine receptors (naturally occurring or recombinant), fragments thereof, mutein receptors, and antibodies, along with compounds identified as having binding affinity to the receptors or antibodies, should be useful in the treatment of conditions exhibiting abnormal expression of the receptors of their ligands. Such abnormality will typically be manifested by immunological disorders. Additionally, this invention should provide therapeutic value in various diseases or disorders associated with abnormal expression or abnormal triggering of response to the ligand. For example, the IL-1 ligands have been suggested to be involved in morphologic development, e.g., dorso-ventral polarity determination, and immune responses, particularly the primitive innate responses. See, e.g., Sun, et al. (1991) Eur. J. Biochem. 196:247-254; and Hultmark (1994) Nature 367:116-117.

Recombinant Cytokine receptors, muteins, agonist or antagonist antibodies thereto, or antibodies can be purified and then administered to a patient. These reagents can be combined for therapeutic use with additional active ingredients, e.g., in conventional pharmaceutically acceptable carriers or diluents, along with physiologically innocuous stabilizers and excipients. These combinations can be sterile, e.g., filtered, and placed into dosage forms as by lyophilization in dosage vials or storage in stabilized aqueous preparations. This invention also contemplates use of antibodies or binding fragments thereof which are not complement binding.

Ligand screening using cytokine receptor or fragments thereof can be performed to identify molecules having binding affinity to the receptors. Subsequent biological assays can then be utilized to determine if a putative ligand can provide competitive binding, which can block intrinsic stimulating activity. Receptor fragments can be used as a blocker or antagonist in that it blocks the activity of ligand. Likewise, a compound having intrinsic stimulating activity can activate the receptor and is thus an agonists in that it simulates the activity of ligand, e.g., inducing signaling. This invention further contemplates the therapeutic use of antibodies to cytokine receptors as antagonists.

The quantities of reagents necessary for effective therapy will depend upon many different factors, including means of administration, target site, reagent physiological life, pharmacological life, physiological state of the patient, and other medicants administered. Thus, treatment dosages should be titrated to optimize safety and efficacy. Typically, dosages used in vitro may provide useful guidance in the amounts useful for in situ administration of these reagents. Animal testing of effective doses for treatment of particular disorders will provide further predictive indication of human dosage. Various considerations are described, e.g., in Gilman, et al. (eds. 1990) Goodman and Gilman's: The Pharmacological Bases of Therapeutics, 8th Ed., Pergamon Press; and Remington's Pharmaceutical Sciences, 17th ed. (1990), Mack Publishing Co., Easton, Penn.

Methods for administration are discussed therein and below, e.g., for oral, intravenous, intraperitoneal, or intramuscular administration, transdermal diffusion, and others. Pharmaceutically acceptable carriers will include water, saline, buffers, and other compounds described, e.g., in the Merck Index, Merck & Co., Rahway, New Jersey. Because of the likely high affinity binding, or turnover numbers, between a putative ligand and its receptors, low dosages of these reagents would be initially expected to be effective. And the signaling pathway suggests extremely low amounts of ligand may have effect. Thus, dosage ranges would ordinarily be expected to be in amounts lower than 1 mM concentrations, typically less than about 10 µM concentrations, usually less than about 100 nM, preferably less than about 10 pM (picomolar), and most preferably less than about 1 fM (femtomolar), with an appropriate carrier. Slow release formulations, or slow release apparatus will often be utilized for continuous administration.

Cytokine receptors, fragments thereof, and antibodies or its fragments, antagonists, and agonists, may be administered directly to the host to be treated or, depending on the size of the compounds, it may be desirable to conjugate them to carrier proteins such as ovalbumin or serum albumin prior to their administration. Therapeutic formulations may be administered in many conventional dosage formulations. While it is possible for the active ingredient to be administered alone, it is preferable to present it as a pharmaceutical formulation. Formulations comprise at least one active ingredient, as defined above, together with one or more acceptable carriers thereof. Each carrier must be both pharmaceutically and physiologically acceptable in the sense of being compatible with the other ingredients and not injurious to the patient. Formulations include those suitable for oral, rectal, nasal, or parenteral (including subcutaneous, intramuscular, intravenous and intradermal) administration. The formulations may conveniently be presented in unit dosage form and may be prepared by methods well known in the art of pharmacy. See, e.g., Gilman, et al. (eds. 1990) Goodman and Gilman's: The Pharmacological Bases of Therapeutics, 8th Ed., Pergamon Press; and Remington's Pharmaceutical Sciences, 17th ed. (1990), Mack Publishing Co., Easton, Penn.; Avis, et al. (eds. 1993) Pharmaceutical Dosage Forms: Parenteral Medications Dekker; NY; Lieberman, et al. (eds. 1990) Pharmaceutical Dosage Forms: Tablets Dekker, NY; and Lieberman, et al. (eds. 1990) Pharmaceutical Dosage Forms: Disperse Systems Dekker, NY. The therapy of this invention may be combined with or used in association with other therapeutic agents, particularly agonists or antagonists of other cytokine receptor family members.

### IX. Screening

Drug screening using DCRS2 or fragments thereof can be performed to identify compounds having binding affinity to the receptor subunit, including isolation of associated components. Subsequent biological assays can then be utilized to determine if the compound has intrinsic stimulating activity and is therefore a blocker or antagonist in that it blocks the activity of the ligand. Likewise, a compound having intrinsic stimulating activity can activate the receptor and is thus an agonist in that it simulates the activity of a cytokine ligand. This invention further contemplates the therapeutic use of antibodies to the receptor as cytokine agonists or antagonists.

Similarly, complexes comprising multiple proteins may be used to screen for ligands or reagents capable of recognizing the complex. Most cytokine receptors comprise at least two subunits, which may be the same, or distinct. Alternatively, the transmembrane receptor may bind to a complex comprising a cytokine-like ligand associated with another soluble protein serving, e.g., as a second receptor subunit.

One method of drug screening utilizes eukaryotic or prokaryotic host cells which are stably transformed with recombinant DNA molecules expressing the DCRS2 in combination with another cytokine receptor subunit. Cells may be isolated which express a receptor in isolation from other functional receptors. Such cells, either in viable or fixed form, can be used for standard antibody/antigen or ligand/receptor binding assays. See also, Parce, et al. (1989) Science 246:243-247; and Owicki, et al. (1990) Proc. Nat'l Acad. Sci. USA 87:4007-4011, which describe sensitive methods to detect cellular responses. Competitive assays are particularly useful, where the cells (source of putative ligand) are contacted and incubated with a labeled receptor or antibody having known binding affinity to the ligand, such as ¹²⁵I-antibody, and a test sample whose binding affinity to the binding composition is being measured. The bound and free labeled binding compositions are then separated to assess the degree of ligand binding. The amount of test compound bound is inversely proportional to the amount of labeled receptor binding to the known source. Many techniques can be used to separate bound from free ligand to assess the degree of ligand binding. This separation step could typically involve a procedure such as adhesion to filters followed by washing, adhesion to plastic followed by washing, or centrifugation of the cell membranes. Viable cells could also be used to screen for the effects of drugs on cytokine mediated functions, e.g., second messenger levels, i.e., Ca⁺⁺; cell proliferation; inositol phosphate pool changes; and others. Some detection methods allow for elimination of a separation step, e.g., a proximity sensitive detection system. Calcium sensitive dyes will be useful for detecting Ca⁺⁺ levels, with a fluorimeter or a fluorescence cell sorting apparatus.

### X. Ligands

The descriptions of the DCRS2 herein provides means to identify ligands, as described above. Such ligand should bind specifically to the respective receptor with reasonably high affinity. Various constructs are made available which allow either labeling of the receptor to detect its ligand. For example, directly labeling cytokine receptor, fusing onto it markers for secondary labeling, e.g., FLAG or other epitope tags, etc., will allow detection of receptor. This can be histological, as an affinity method for biochemical purification, or labeling or selection in an expression cloning approach. A two-hybrid selection system may also be applied making appropriate constructs with the available cytokine receptor sequences. See, e.g., Fields and Song (1989) Nature 340:245-246.

The broad scope of this invention is best understood with reference to the following examples, which are not intended to limit the inventions to the specific embodiments.

### EXAMPLES

### I. General Methods

Some of the standard methods are described or referenced, e.g., in Maniatis, et al. (1982) molecular Cloning. A Laboratory Manual, Cold Spring Harbor Laboratory, Cold Spring Harbor Press; Sambrook, et al. (1989) Molecular Cloning: A Laboratory Manual, (2d ed.), vols. 1-3, CSH Press, NY; or Ausubel, et al. (1987 and Supplements) Current Protocols in Molecular Biology, Greene/Wiley, New York. Methods for protein purification include such methods as ammonium sulfate precipitation, column chromatography, electrophoresis, centrifugation, crystallization, and others. See, e.g., Ausubel, et al. (1987 and periodic supplements); Coligan, et al. (ed. 1996) and periodic supplements, Current Protocols In Protein Science Greene/Wiley, New York; Deutscher (1990) "Guide to Protein Purification" in Methods in Enzymology, vol. 182, and other volumes in this series; and manufacturer's literature on use of protein purification products, e.g., Pharmacia, Piscataway, N.J., or Bio-Rad, Richmond, CA. Combination with recombinant techniques allow fusion to appropriate segments, e.g., to a FLAG sequence or an equivalent which can be fused via a protease-removable sequence. See, e.g., Hochuli (1990) "Purification of Recombinant Proteins with Metal Chelate Absorbent" in Setlow (ed.) Genetic Engineering, Principle and Methods 12:87-98, Plenum Press, N.Y.; and Crowe, et al. (1992) QIAexpress: The High Level Expression & Protein Purification System QUIAGEN, Inc., Chatsworth, CA.

Computer sequence analysis is performed, e.g., using available software programs, including those from the GCG (U. Wisconsin) and GenBank sources. Public sequence databases were also used, e.g., from GenBank and others.

Many techniques applicable to IL-10 receptors may be applied to the DCRS2, as described, e.g., in USSN 08/110,683 (IL-10 receptor).

### II. Computational Analysis

Human sequences related to cytokine receptors were identified from genomic sequence database using, e.g., the BLAST server (Altschul, et al. (1994) Nature Genet. 6:119-129). Standard analysis programs may be used to evaluate structure, e.g., PHD (Rost and Sander (1994) Proteins 19:55-72) and DSC (King and Sternberg (1996) Protein Sci. 5:2298-2310). Standard comparison software includes, e.g., Altschul, et al. (1990) J. Mol. Biol. 215:403-10; Waterman (1995) Introduction to Computational Biology: Maps, Sequences, and Genomes Chapman & Hall; Lander and Waterman (eds. 1995) Calculating the Secrets of Life: Applications of the Mathematical Sciences in Molecular Biology National Academy Press; and Speed and Waterman (eds. 1996) Genetic Mapping and DNA Sequencing (IMA Volumes in Mathematics and Its Applications, Vol 81) Springer Verlag.

### III. Cloning of full-length DCRS2 cDNAs; Chromosomal localization

PCR primers derived from the DCRS2 sequence are used to probe a human cDNA library. Sequences may be derived, e.g., from Table 1, preferably those adjacent the ends of sequences. Full length cDNAs for primate, rodent, or other species DCRS2 are cloned, e.g., by DNA hybridization screening of λgt10 phage. PCR reactions are conducted using T. aquaticus Taqplus DNA polymerase (Stratagene) under appropriate conditions.

Chromosome spreads are prepared. In situ hybridization i's performed on chromosome preparations obtained from phytohemagglutinin-stimulated human lymphocytes cultured for 72 h. 5-bromodeoxyuridine was added for the final seven hours of culture (60 µg/ml of medium), to ensure a posthybridization chromosomal banding of good quality.

A PCR fragment, amplified with the help of primers, is cloned into an appropriate vector. The vector is labeled by nick-translation with ³H. The radiolabeled probe is hybridized to metaphase spreads at final concentration of 200 ng/ml of hybridization solution as described in Mattei, et al. (1985) Hum. Genet. 69:327-331.

After coating with nuclear track emulsion (KODAK NTB₂), slides are exposed. To avoid any slipping of silver grains during the banding procedure, chromosome spreads are first stained with buffered Giemsa solution and metaphase photographed. R-banding is then performed by the fluorochrome-photolysis-Giemsa (FPG) method and metaphases rephotographed before analysis.

Similar appropriate methods are used for other species.

### IV. Localization of DCRS2 mRNA

Human multiple tissue (Cat# 1, 2) and cancer cell line blots (Cat# 7757-1), containing approximately 2 µg of poly(A)⁺ RNA per lane, are purchased from Clontech (Palo Alto, CA). Probes are radiolabeled with [α-³²P] dATP, e.g., using the Amersham Rediprime random primer labeling kit (RPN1633). Prehybridization and hybridizations are performed, e.g., at 65° C in 0.5 M Na₂HPO₄, 7% SDS, 0.5 M EDTA (pH 8.0). High stringency washes are conducted, e.g., at 65° C with two initial washes in 2 x SSC, 0.1% SDS for 40 min followed by a subsequent wash in 0.1 x SSC, 0.1% SDS for 20 min. Membranes are then exposed at -70° C to X-Ray film (Kodak) in the presence of intensifying screens. More detailed studies by cDNA library Southerns are performed with selected appropriate human DCRS2 clones to examine their expression in hemopoietic or other cell subsets.

Alternatively, two appropriate primers are selected from Table 1. RT-PCR is used on an appropriate mRNA sample selected for the presence of message to produce a cDNA, e.g., a sample which expresses the gene.

Full length clones may be isolated by hybridization of cDNA libraries from appropriate tissues pre-selected by PCR signal. Northern blots can be performed.

Message for genes encoding DCRS2 will be assayed by appropriate technology, e.g., PCR, immunoassay, hybridization, or otherwise. Tissue and organ cDNA preparations are available, e.g., from Clontech, Mountain View, CA. Identification of sources of natural expression are useful, as described. And the identification of functional receptor subunit pairings will allow for prediction of what cells express the combination of receptor subunits which will result in a physiological responsiveness to each of the cytokine ligands.

For mouse distribution, e.g., Southern Analysis can be performed: DNA (5 µg) from a primary amplified cDNA library was digested with appropriate restriction enzymes to release the inserts, run on a 1% agarose gel and transferred to a nylon membrane (Schleicher and Schuell, Keene, NH).

Samples for mouse mRNA isolation may include: resting mouse fibroblastic L cell line (C200); Braf:ER (Braf fusion to estrogen receptor) transfected cells, control (C201); T cells, TH1 polarized (Mel14 bright, CD4+ cells from spleen, polarized for 7 days with IFN-γ and anti IL-4; T200); T cells, TH2 polarized (Mel14 bright, CD4+ cells from spleen, polarized for 7 days with IL-4 and anti-IFN-γ; T201); T cells, highly TH1 polarized (see Openshaw, et al. (1995) J. Exp. Med. 182:1357-1367; activated with anti-CD3 for 2, 6, 16 h pooled; T202); T cells, highly TH2 polarized (see Openshaw, et al: (1995) J. Exp. Med. 182:1357-1367; activated with anti-CD3 for 2, 6, 16 h pooled; T203); CD44- CD25+ pre T cells, sorted from thymus (T204); TH1 T cell clone D1.1, resting for 3 weeks after last stimulation with antigen (T205); TH1 T cell clone D1.1, 10 µg/ml ConA stimulated 15 h (T206); TH2 T cell clone CDC35, resting for 3 weeks after last stimulation with antigen (T207) ; TH2 T cell clone CDC35, 10 µg/ml ConA stimulated 15 h (T208); Mel14+ naive T cells from spleen, resting (T209); Mel14+ T cells, polarized to Th1 with IFN-y/IL-12/anti-IL-4 for 6, 12, 24 h pooled (T210); Mel14+ T cells, polarized to Th2 with IL-4/anti-IFN-γ for 6, 13, 24 h pooled (T211) ; unstimulated mature B cell leukemia cell line A20 (B200); unstimulated B cell line CH12 (B201); unstimulated large B cells from spleen (B202) ; B cells from total spleen, LPS activated (B203) ; metrizamide enriched dendritic cells from spleen, resting (D200); dendritic cells from bone marrow, resting (D201) ; monocyte cell line RAW 264.7 activated with LPS 4 h (M200) ; bone-marrow macrophages derived with GM and M-CSF (M201) ; macrophage cell line J774, resting (M202); macrophage cell line J774 + LPS + anti-IL-10 at 0.5, 1, 3, 6, 12 h pooled (M203) ; macrophage cell line J774 + LPS + IL-10 at 0.5, 1, 3, 5, 12 h pooled (M204) ; aerosol challenged mouse lung tissue, Th2 primers, aerosol OVA challenge 7, 14, 23 h-pooled (see Garlisi, et al. (1995) Clinical Immunology and Immunopathology 75:75-83; X206) ; Nippostrongulus-infected lung tissue (see Coffman, et al. (1989) Science 245:308-310; X200); total adult lung, normal (O200) ; total lung, rag-1 (see Schwarz, et al. (1993) Immunodeficiency 4:249-252; 0205); IL-10 K.O. spleen (see Kuhn, et al. (1991) Cell 75:263-274; X201); total adult spleen, normal (O201); total spleen, rag-1 (O207); IL-10 K.O. Peyer's patches (O202); total Peyer's patches, normal (O210); IL-10 K.O. mesenteric lymph nodes (X203); total mesenteric lymph nodes, normal (O211); IL-10 K.O. colon (X203); total colon, normal (O212); NOD mouse pancreas (see Makino, et al. (1980) Jikken Dobutsu 29:1-13; X205); total thymus, rag-1 (O208); total kidney, rag-1 (O209); total heart, rag-1 (O202); total brain, rag-1 (O203); total testes, rag-1 (O204); total liver, rag-1 (O206); rat normal joint tissue (O300); and rat arthritic joint tissue (X300).

Samples for human mRNA isolation may include: peripheral blood mononuclear cells (monocytes, T cells, NK cells, granulocytes, B cells), resting (T100); peripheral blood mononuclear cells, activated with anti-CD3 for 2, 6, 12 h pooled (T101); T cell, TH0 clone Mot 72, resting (T102); T cell, TH0 clone Mot 72, activated with anti-CD28 and anti-CD3 for 3, 6, 12 h pooled (T103); T cell, TH0 clone Mot 72, anergic treated with specific peptide for 2, 7, 12 h pooled (T104) ; T cell, TH1 clone HY06, resting (T107); T cell, TH1 clone HY06, activated with anti-CD28 and anti-CD3 for 3, 6, 12 h pooled (T108); T cell, TH1 clone HY06, anergic treated with specific peptide for 2, 6, 12 h pooled (T109); T cell, TH2 clone HY935, resting (t110) ; T cell, TH2 clone HY935, activated with anti-CD28 and anti-CD3 for 2, 7, 12 h pooled (T111); T cells CD4+CD45RO- T cells polarized 27 days in anti-CD28, IL-4, and anti IFN-γ, TH2 polarized, activated with anti-CD3 and anti-CD28 4 h (T116); T cell tumor lines Jurkat and Hut78, resting (T117); T cell clones, pooled AD130.2, Tc783.12, Tc783.13, Tc783.58, Tc782.69, resting (T118); T cell random γδ T cell clones, resting (T119) ; Splenocytes, resting (B100); Splenocytes, activated with anti-CD40 and IL-4 (B101); B cell EBV lines pooled WT49, RSB, JY, CVIR, 721.221, RM3, HSY, resting (B102) ; B cell line JY, activated with PMA and ionomycin for 1, 6 h pooled (B103); NK 20 clones pooled, resting (K100) ; NK 20 clones pooled, activated with PMA and ionomycin for 6 h (K101) ; NKL clone, derived from peripheral blood of LGL leukemia patient, IL-2 treated (K106); NK cytotoxic clone 640-A30-1, resting (K107); hematopoietic precursor line TF1, activated with PMA and ionomycin for 1, 6 h pooled (C100) ; U937 premonocytic line, resting (M100); U937 premonocytic line, activated with PMA and ionomycin for 1, 6 h pooled (M101) ; elutriated monocytes, activated with LPS, IFNγ, anti-IL-10 for 1, 2, 6, 12, 24 h pooled (M102) ; elutriated monocytes, activated with LPS, IFNγ, IL-10 for 1, 2, 6, 12, 24 h pooled (M103); elutriated monocytes, activated with LPS, IFNγ, anti-IL-10 for 4, 16 h pooled (M106); elutriated monocytes, activated with LPS, IFNγ, IL-10 for 4, 16 h pooled (M107) ; elutriated monocytes, activated LPS for 1 h (M108); elutriated monocytes, activated LPS for 6 h (M109) ; DC 70% CD1a+, from CD34+ GM-CSF, TNFα 12 days, resting (D101); DC 70% CD1a+, from CD34+ GM-CSF, TNFα 12 days, activated with-PMA and ionomycin for 1 hr (D102) ; DC 70% CD1a+, from CD34 + GM-CSF, TNFα 12 days, activated with PMA and ionomycin for 6 hr (D103); DC 95% CD1a+, from CD34+ GM-CSF, TNFα 12 days FACS sorted, activated with PMA and ionomycin for 1, 6 h pooled (D104); DC 95% CD14+, ex CD34+ GM-CSF, TNFα 12 days FACS sorted, activated with PMA and ionomycin 1, 6 hr pooled (D105); DC CD1a+ CD86+, from CD34+ GM-CSF, TNFα 12 days FACS sorted, activated with PMA and ionomycin for 1, 6 h pooled (D106) ; DC from monocytes GM-CSF, IL-4 5 days, resting (D107) ; DC from monocytes GM-CSF, IL-4 5 days, resting (D108); DC from monocytes GM-CSF, IL-4 5 days, activated LPS 4, 16 h pooled (D109) ; DC from monocytes GM-CSF, IL-4 5 days, activated TNFα, monocyte supe for 4, 16 h pooled (D110); leiomyoma L11 benign tumor (X101); normal myometrium M5 (O115); malignant leiomyosarcoma GS1 (X103) ; lung fibroblast sarcoma line MRC5, activated with PMA and ionomycin for 1, 6 h pooled (C101); kidney epithelial carcinoma cell line CHA, activated with PMA and ionomycin for 1, 6 h pooled (C102); kidney fetal 28 wk male (O100); lung fetal 28 wk male (O101); liver fetal 28 wk male (O102 ) ; heart fetal 28 wk male (O103) ; brain fetal 28 wk male (O104) ; gallbladder fetal 28 wk male (O106); small intestine fetal 28 wk male (O107); adipose tissue fetal 28 wk male (O108); ovary fetal 25 wk female (O109) ; uterus fetal 25 wk female (O110); testes fetal 28 wk male (O111); spleen fetal 28 wk male (O112); adult placenta 28 wk (O113); and tonsil inflamed, from 12 year old (X100).

Similar samples may isolated in other species for evaluation.

### V. Cloning of species counterparts of DCRS2

Various strategies are used to obtain species counterparts of the DCRS2, preferably from other primates or rodents. One method is by cross hybridization using closely related species DNA probes. It may be useful to go into evolutionarily similar species as intermediate steps. Another method is by using specific PCR primers based on the identification of blocks of similarity or difference between genes, e.g., areas of highly conserved or nonconserved polypeptide or nucleotide sequence.

### VI. Production of mammalian DCRS2 protein

An appropriate, e.g., GST, fusion construct is engineered for expression, e.g., in E. coli. For example, a mouse IGIF pGex plasmid is constructed and transformed into E. coli. Freshly transformed cells are grown, e.g., in LB medium containing 50 µg/ml ampicillin and induced with IPTG (Sigma, St. Louis, MO). After overnight induction, the bacteria are harvested and the pellets containing the DCRS2 protein are isolated. The pellets are homogenized, e.g., in TE buffer (50 mM Tris-base pH 8.0, 10 mM EDTA and 2 mM pefabloc) in 2 liters. This material is passed through a microfluidizer (Microfluidics, Newton, MA) three times. The fluidized supernatant is spun down on a Sorvall GS-3 rotor for 1 h at 13,000 rpm. The resulting supernatant containing the cytokine receptor protein is filtered and passed over a glutathione-SEPHAROSE column equilibrated in 50 mM Tris-base pH 8.0. The fractions containing the DCRS2-GST fusion protein are pooled and cleaved, e.g., with thrombin (Enzyme Research Laboratories, Inc., South Bend, IN). The cleaved pool is then passed over a Q-SEPHAROSE column equilibrated in 50 mM Tris-base. Fractions containing DCRS2 are pooled and diluted in cold distilled H₂O, to lower the conductivity, and passed back over a fresh Q-Sepharose column, alone or in succession with an immunoaffinity antibody column. Fractions containing the DCRS2 protein are pooled, aliquoted, and stored in the -70° C freezer.

Comparison of the CD spectrum with cytokine receptor protein may suggest that the protein is correctly folded. See Hazuda, et al. (1969) J. Biol. Chem. 264:1689-1693.

### VII. Preparation of antibodies specific for DCRS2

Inbred Balb/c mice are immunized intraperitoneally with recombinant forms of the protein, e.g., purified DCRS2 or stable transfected NIH-3T3 cells. Animals are boosted at appropriate time points with protein, with or without additional adjuvant, to further stimulate antibody production. Serum is collected, or hybridomas produced with harvested spleens.

Alternatively, Balb/c mice are immunized with cells transformed with the gene or fragments thereof, either endogenous or exogenous cells, or with isolated membranes enriched for expression of the antigen. Serum is collected at the appropriate time, typically after numerous further administrations. Various gene therapy techniques may be useful, e.g., in producing protein in situ, for generating an immune response. Serum or antibody preparations may be cross-absorbed or immunoselected to prepare substantially purified antibodies of defined specificity and high affinity.

Monoclonal antibodies may be made. For example, splenocytes are fused with an appropriate fusion partner and hybridomas are selected in growth medium by standard procedures. Hybridoma supernatants are screened for the presence of antibodies, which bind to the DCRS2, e.g., by ELISA or other assay. Antibodies which specifically recognize specific DCRS2 embodiments may also be selected or prepared.

In another method, synthetic peptides or purified protein are presented to an immune system to generate monoclonal or polyclonal antibodies. See, e.g., Coligan (ed. 1991) Current Protocols in Immunology Wiley/Greene; and Harlow and Lane (1989) Antibodies: A Laboratory Manual Cold Spring Harbor Press. In appropriate situations, the binding reagent is either labeled as described above, e.g., fluorescence or otherwise, or immobilized to a substrate for panning methods. Nucleic acids may also be introduced into cells in an animal to produce the antigen, which serves to elicit an immune response. See, e.g., Wang, et al. (1993) Proc. Nat'l. Acad. Sci. 90:4156-4160; Barry, et al. (1994) BioTechniques 16:616-619; and Xiang, et al. (1995) Immunity 2: 129-135.

### VIII. Production of fusion proteins with DCRS2

Various fusion constructs are made with DCRS2. A portion of the appropriate gene is fused to an epitope tag, e.g., a FLAG tag, or to a two hybrid system construct. See, e.g., Fields and Song (1989) Nature 340:245-246.

The epitope tag may be used in an expression cloning procedure with defection with anti-FLAG antibodies to detect a binding partner, e.g., ligand for the respective cytokine receptor. The two hybrid system may also be used to isolate' proteins which specifically bind to DCRS2.

### IX. Structure activity relationship

Information en the criticality of particular residues is determined using standard procedures and analysis. Standard mutagenesis analysis is performed, e.g., by generating many different variants at determined positions, e.g., at the positions identified above, and evaluating biological activities of the variants. This may be performed to the extent of determining positions which modify activity, or to focus on specific positions to determine the residues which can be substituted to either retain, block, or modulate biological activity.

Alternatively, analysis of natural variants can indicate what positions tolerate natural mutations. This may result from populational analysis of variation among individuals, or across strains or species. Samples from selected individuals are analyzed, e.g., by PCR analysis and sequencing. This allows evaluation of population polymorphisms.

### X. Isolation of a ligand for DCRS2

A cytokine receptor can be used as a specific binding reagent to identify its binding partner, by taking advantage of its specificity of binding, much like an antibody would be used. The binding receptor may be a heterodimer of receptor subunits; or may involve, e.g., a complex of the DCRS2 with another subunit. A binding reagent is either labeled as described above, e.g., fluorescence or otherwise, or immobilized to a substrate for panning methods.

The binding composition is used to screen an expression library made from a cell line which expresses a binding partner, i.e., ligand, preferably membrane associated. Standard staining techniques are used to detect or sort surface expressed ligand, or surface expressing transformed cells are screened by panning. Screening of intracellular expression is performed by various staining or immunofluorescence procedures. See also McMahan, et al. (1991) EMBO J. 10:2821-2832.

For example, on day 0, precoat 2-chamber permanox slides with 1 ml per chamber of fibronectin, 10 ng/ml in PBS, for 30 min at room temperature. Rinse once with PBS. Then plate COS cells at 2-3 x 10⁵ cells per chamber in 1.5 ml of growth media. Incubate overnight at 37° C.

On day 1 for each sample, prepare 0.5 ml of a solution of 66 µg/ml DEAE-dextran, 66 µM chloroquine, and 4 µg DNA in serum free DME. For each set, a positive control is prepared, e.g., of DCRS2-FLAG cDNA at 1 and 1/200 dilution, and a negative mock. Rinse cells with serum free DME. Add the DNA solution and incubate 5 hr at 37° C. Remove the medium and add 0.5 ml 10% DMSO in DME for 2.5 min. Remove and wash once with DME. Add 1.5 ml growth medium and incubate overnight.

On day 2, change the medium. On days 3 or 4, the cells are fixed and stained. Rinse the cells twice with Hank's Buffered Saline Solution (HBSS) and fix in 4% paraformaldehyde (PFA)/glucose for 5 min. Wash 3X with HBSS. The slides may be stored at -80° C after all liquid is removed. For each chamber, 0.5 ml incubations are performed as follows. Add HBSS/saponin (0.1%) with 32 µl/ml of 1 M NaN₃ for 20 min. Cells are then washed with HBSS/saponin 1X. Add appropriate DCRS2 or DCRS2/antibody complex to cells and incubate for 30 min. Wash cells twice with HBSS/saponin. If appropriate, add first antibody for 30 min. Add second antibody, e.g., Vector antimouse antibody, at 1/200 dilution, and incubate for 30 min. Prepare ELISA solution, e.g., Vector Elite ABC horseradish peroxidase solution, and preincubate for 30 min. Use, e.g., 1 drop of solution A (avidin) and 1 drop solution B (biotin) per 2.5 ml HBSS/saponin. Wash cells twice with HBSS/saponin. Add ABC HRP solution and incubate for 30 min. Wash cells twice with HBSS, second wash for 2 min, which closes cells. Then add Vector diaminobenzoic acid (DAB) for 5 to 10 min. Use 2 drops of buffer plus 4 drops DAB plus 2 drops of H₂O₂ per 5 ml of glass distilled water. Carefully remove chamber and rinse slide in water. Air dry for a few minutes, then add 1 drop of Crystal Mount and a cover slip. Bake for 5 min at 85-90° C.

Evaluate positive staining of pools and progressively subclone to isolation of single genes responsible for the binding.

Alternatively, receptor reagents are used to affinity purify or sort out cells expressing a putative ligand. See, e.g., Sambrook, et al. or Ausubel, et al.

Another strategy is to screen for a membrane bound receptor by panning. The receptor cDNA is constructed as described above. Immobilization may be achieved by use of appropriate antibodies which recognize, e.g., a FLAG sequence of a DCRS2 fusion construct, or by use of antibodies raised against the first antibodies. Recursive cycles of selection and amplification lead to enrichment of appropriate clones and eventual isolation of receptor expressing clones.

Phage expression libraries can be screened by mammalian DCRS2. Appropriate label techniques, e.g., anti-FLAG antibodies, will allow specific labeling of appropriate clones.

### SEQUENCE SUBMISSION

SEQ ID NO: 1 is primate DCRS2 nucleotide sequence.
SEQ ID NO: 2 is primate DCRS2 polypeptide sequence.
SEQ ID NO: 3 is primate DCRS2 reverse translation.
SEQ ID NO: 4 is primate NR6 polypeptide sequence.
SEQ ID NO: 5 is rodent NR6 polypeptide sequence.
SEQ ID NO: 6 is primate p40 polypeptide sequence.
SEQ ID NO: 7 is rodent p40 polypeptide sequence.
SEQ ID NO: 8 is rodent Ebi3 polypeptide sequence.
SEQ ID NO: 9 is primate Ebi3 polypeptide sequence.
SEQ ID NO: 10 is rodent IL-11 receptor subunit alpha polypeptide sequence.
SEQ ID NO: 11 is primate IL-11 receptor subunit alpha polypeptide sequence.
SEQ ID NO: 12 is primate IL-6 receptor subunit alpha polypeptide sequence.
SEQ ID NO: 13 is rodent IL-6 receptor subunit alpha polypeptide sequence.

## Claims

1. An isolated polypeptide comprising at least 70% amino acid sequence homology to the mature amino acid sequence (residues 1-361) of SEQ ID NO: 2.

2. The polypeptide of Claim 1 wherein the polypeptide comprises at least 90% amino acid sequence homology to the mature amino acid sequence (residues 1-361) of SEQ ID NO: 2.

3. The polypeptide of Claim 2 wherein the polypeptide comprises at least 98% amino acid sequence homology to the mature amino acid sequence (residues 1-361) of SEQ ID NO: 2.

4. The polypeptide of Claim 3 wherein the polypeptide comprises the sequence of residues 1-361 of SEQ ID NO: 2.

5. The polypeptide of Claim 1 wherein the polypeptide comprises at least 70% amino acid sequence homology to the amino acid sequence of SEQ ID NO: 2.

6. The polypeptide of Claim 5 wherein the polypeptide comprises at least 90% amino acid sequence homology to the amino acid sequence of SEQ ID NO: 2.

7. The polypeptide of Claim 6 wherein the polypeptide comprises the sequence of SEQ ID NO: 2.

8. A pharmaceutical formulation comprising:
a) the polypeptide of Claim 1; and
b) a pharmaceutically acceptable carrier or diluent.

9. A composition of matter comprising a fusion protein comprising the polypeptide of Claim 1 and a detection or purification tag.

10. An isolated or recombinant nucleic acid encoding the polypeptide of Claim 1.

11. The nucleic acid of Claim 10 comprising the sequence of residues 70-1152 of SEQ ID NO: 1.

12. An expression vector comprising the nucleic acid of Claim 10.

13. A cell comprising the expression vector of Claim 12.

14. The cell of Claim 13, wherein said cell is:
a) a bacterial cell;
b) a yeast cell;
c) an insect cell; or
d) a mammalian cell.

15. The nucleic acid of Claim 10 comprising a detectable label.

16. A nucleic acid that hybridizes under wash conditions of 30 minutes at 45°C and 500 mM salt to the coding portion of SEQ ID NO: 1.

17. The nucleic acid of Claim 16, wherein said wash conditions are 30 minutes at 55°C and 150 mM salt.

18. A nucleic acid comprising at least 71% nucleotide sequence identity with the coding portion (residues 1-1152) of SEQ ID NO: 1.

19. The nucleic acid of Claim 18 comprising at least 80% sequence identity with coding portion (residues 1-1152) of SEQ ID NO: 1.

20. The nucleic acid of Claim 19 comprising at least 95% sequence identity with coding portion (residues 1-1152) of SEQ ID NO: 1.

21. The nucleic acid of Claim 20 comprising the coding portion (residues 1-1152) of SEQ ID NO: 1.

22. A binding compound comprising an antigen binding site from an antibody that specifically binds to a polypeptide comprising the mature amino acid sequence (residues 1-361) of SEQ ID NO: 2.

23. A binding compound comprising an antigen binding site from an antibody that specifically binds to a polypeptide consisting of the mature amino acid sequence (residues 1-361) of SEQ ID NO: 2.

24. The binding compound of Claim 22 or Claim 23 comprising an antibody fragment selected from the group consisting of an Fv fragment, an Fab fragment, and an Fab2 fragment.

25. The binding compound of Claim 22 or Claim 23 wherein the binding compound is detectably labeled.

26. A pharmaceutical formulation comprising:
a) the binding compound of Claim 22 or Claim 23; and
b) a pharmaceutically acceptable carrier or diluent,
wherein the binding compound is an antibody having agonistic or antagonistic activity.

27. An in vitro method of producing an antigen:antibody complex, comprising:
a) contacting the polypeptide of Claim 1 with the binding compound of Claim 22 or Claim 23; and
b) allowing said complex to form.

28. A complex comprising:
a) the polypeptide of Claim 1; and
b) the binding compound of Claim 22 or Claim 23.

29. The binding compound of Claim 22 or Claim 23, wherein the antibody is a chimeric antibody.

30. The binding compound of Claim 22 or Claim 23, wherein the antibody is a humanized antibody.

31. The binding compound of Claim 22 or Claim 23, wherein the antibody is a human antibody.

32. Use of the binding compound of Claim 22 or Claim 23 in the manufacture of a medicament, wherein the binding compound is an antibody having agonistic or antagonistic activity.

33. A composition comprising the binding compound of Claim 22 or Claim 23 for use as a medicament, wherein the binding compound is an antibody having agonistic or antagonistic activity.

## Patentansprüche

1. Isoliertes Polypeptid, das mindestens 70 % Aminosäuresequenzhomologie zu der reifen Aminosäuresequenz (Reste 1-361) von SEQ ID NO: 2 umfasst.

2. Polypeptid nach Anspruch 1, wobei das Polypeptid mindestens 90 % Aminosäuresequenzhomologie zu der reifen Aminosäuresequenz (Reste 1-361) von SEQ ID NO: 2 umfasst.

3. Polypeptid nach Anspruch 2, wobei das Polypeptid mindestens 98 % Aminosäuresequenzhomologie zu der reifen Aminosäuresequenz (Reste 1-361) von SEQ ID NO: 2 umfasst.

4. Polypeptid nach Anspruch 3, wobei das Polypeptid die Sequenz der Reste 1-361 von SEQ ID NO: 2 umfasst.

5. Polypeptid nach Anspruch 1, wobei das Polypeptid mindestens 70 % Aminosäuresequenzhomologie zu der Aminosäuresequenz von SEQ ID NO: 2 umfasst.

6. Polypeptid nach Anspruch 5, wobei das Polypeptid mindestens 90 % Aminosäuresequenzhomologie zu der Aminosäuresequenz von SEQ ID NO: 2 umfasst.

7. Polypeptid nach Anspruch 6, wobei das Polypeptid die Sequenz von SEQ ID NO: 2 umfasst.

8. Pharmazeutische Formulierung, umfassend:
a) das Polypeptid nach Anspruch 1 und
b) einen pharmazeutisch annehmbaren Träger oder ein pharmazeutisch annehmbares Verdünnungsmittel.

9. Stoffzusammensetzung, die ein Fusionsprotein umfasst, das das Polypeptid nach Anspruch 1 und ein Nachweis- oder Reinigungs-Tag umfasst.

10. Isolierte oder rekombinante Nukleinsäure, die für das Polypeptid nach Anspruch 1 kodiert.

11. Nukleinsäure nach Anspruch 10, die die Sequenz der Reste 70-1152 von SEQ ID NO: 1 umfasst.

12. Expressionsvektor, der die Nukleinsäure nach Anspruch 10 umfasst.

13. Zelle, die den Expressionsvektor nach Anspruch 12 umfasst.

14. Zelle nach Anspruch 13, wobei die Zelle:
a) eine bakterielle Zelle,
b) eine Hefe-Zelle,
c) eine Insekten-Zelle oder
d) eine Säugetier-Zelle
ist.

15. Nukleinsäure nach Anspruch 10, die eine nachweisbare Markierung umfasst.

16. Nukleinsäure, die unter Waschbedingungen von 30 Minuten bei 45°C und 500 mM Salz an den kodierenden Teil von SEQ ID NO: 1 hybridisiert.

17. Nukleinsäure nach Anspruch 16, wobei die Waschbedingungen 30 Minuten bei 55°C und 150 mM Salz sind.

18. Nukleinsäure, die mindestens 71 % Nukleotidsequenzidentität mit dem kodierenden Teil (Reste 1-1152) von SEQ ID NO: 1 umfasst.

19. Nukleinsäure nach Anspruch 18, die mindestens 80 % Sequenzidentität mit dem kodierenden Teil (Reste 1-1152) von SED ID NO: 1 umfasst.

20. Nukleinsäure nach Anspruch 19, die mindestens 95 % Sequenzidentität mit dem kodierenden Teil (Reste 1-1152) von SED ID NO: 1 umfasst.

21. Nukleinsäure nach Anspruch 20, die den kodierenden Teil (Reste 1-1152) von SED ID NO: 1 umfasst.

22. Bindende Verbindung, die eine Antigenbindungsstelle aus einem Antikörper umfasst, der spezifisch an ein Polypeptid bindet, das die reife Aminosäuresequenz (Reste 1-361) von SEQ ID NO: 2 umfasst.

23. Bindende Verbindung, die eine Antigenbindungsstelle aus einem Antikörper umfasst, der spezifisch an ein Polypeptid bindet, welches aus der reifen Aminosäuresequenz (Reste 1-361) von SEQ ID NO: 2 besteht.

24. Bindende Verbindung nach Anspruch 22 oder Anspruch 23, die ein Antikörperfragment umfasst, das ausgewählt ist aus der Gruppe bestehend aus einem Fv-Fragment, einem Fab-Fragment und einem Fab2-Fragment.

25. Bindende Verbindung nach Anspruch 22 oder Anspruch 23, wobei die bindende Verbindung nachweisbar markiert ist.

26. Pharmazeutische Formulierung, umfassend:
a) die bindende Verbindung nach Anspruch 22 oder Anspruch 23 und
b) ein pharmazeutisch annehmbares Verdünnungsmittel oder einen pharmazeutisch annehmbaren Träger,
wobei die bindende Verbindung ein Antikörper ist, der agonistische oder antagonistische Aktivität aufweist.

27. In-vitro-Verfahren zur Herstellung eines Antigen:Antikörper-Komplexes, bei dem man:
a) das Polypeptid nach Anspruch 1 mit der bindenden Verbindung nach Anspruch 22 oder Anspruch 23 in Kontakt bringt und
b) es dem Komplex erlaubt, sich zu bilden.

28. Komplex, umfassend:
a) das Polypeptid nach Anspruch 1 und
b) die bindende Verbindung nach Anspruch 22 oder Anspruch 23.

29. Bindende Verbindung nach Anspruch 22 oder Anspruch 23, wobei der Antikörper ein chimärer Antikörper ist.

30. Bindende Verbindung nach Anspruch 22 oder Anspruch 23, wobei der Antikörper ein humanisierter Antikörper ist.

31. Bindende Verbindung nach Anspruch 22 oder Anspruch 23, wobei der Antikörper ein humaner Antikörper ist.

32. Verwendung der bindenden Verbindung nach Anspruch 22 oder Anspruch 23 bei der Herstellung eines Medikamentes, wobei die bindende Verbindung ein Antikörper mit agonistischer oder antagonistischer Aktivität ist.

33. Zusammensetzung, die die bindende Verbindung nach Anspruch 22 oder Anspruch 23 umfasst, zur Verwendung als Medikament, wobei die bindende Verbindung ein Antikörper mit agonistischer oder antagonistischer Aktivität ist.

## Revendications

1. Polypeptide isolé, qui comprend une séquence d'acides aminés homologue, pour au moins 70 %, à la séquence d'acides aminés mature (résidus n° 1 à 361) dérivée de la SEQ ID N° 2.

2. Polypeptide selon la revendication 1, lequel polypeptide comprend une séquence d'acides aminés homologue, pour au moins 90 %, à la séquence d'acides aminés mature (résidus n° 1 à 361) dérivée de la SEQ ID N° 2.

3. Polypeptide selon la revendication 2, lequel polypeptide comprend une séquence d'acides aminés homologue, pour au moins 98 %, à la séquence d'acides aminés mature (résidus n° 1 à 361) dérivée de la SEQ ID N° 2.

4. Polypeptide selon la revendication 3, lequel polypeptide comprend la séquence des résidus n° 1 à 361 de la SEQ ID N° 2.

5. Polypeptide selon la revendication 1, lequel polypeptide comprend une séquence d'acides aminés homologue, pour au moins 70 %, à la séquence d'acides aminés donnée en tant que SEQ ID N° 2.

6. Polypeptide selon la revendication 5, lequel polypeptide comprend une séquence d'acides aminés homologue, pour au moins 90 %, à la séquence d'acides aminés donnée en tant que SEQ ID N° 2.

7. Polypeptide selon la revendication 6, lequel polypeptide comprend la séquence donnée en tant que SEQ ID N° 2.

8. Formulation pharmaceutique comprenant :
a) un polypeptide selon la revendication 1, et
b) un véhicule ou diluant pharmaceutiquement acceptable.

9. Composition de matière comportant une protéine de fusion qui comprend un polypeptide selon la revendication 1 et une étiquette de détection ou de purification.

10. Acide nucléique isolé ou recombiné, codant le polypeptide selon la revendication 1.

11. Acide nucléique selon la revendication 10, comprenant la séquence des résidus n° 70 à 1152 de la SEQ ID N° 1.

12. Vecteur d'expression comprenant l'acide nucléique selon la revendication 10.

13. Cellule comprenant un vecteur d'expression selon la revendication 12.

14. Cellule selon la revendication 13, laquelle cellule est :
a) une cellule bactérienne,
b) une cellule de levure,
c) une cellule d'insecte, ou
d) une cellule de mammifère.

15. Acide nucléique selon la revendication 10, comprenant un marqueur détectable.

16. Acide nucléique qui s'hybride à la partie codante de la SEQ ID N° 1, dans les conditions de lavage suivantes : 30 minutes, 45 °C et 500 mM de sel.

17. Acide nucléique selon la revendication 16, pour lequel les conditions de lavage sont les suivantes : 30 minutes, 55 °C et 150 mM de sel.

18. Acide nucléique, qui comprend une identité de séquence de nucléotides, pour au moins 71 %, à la partie codante (résidus n° 1 à 1152) de la SEQ ID N° 1.

19. Acide nucléique selon la revendication 18, qui comprend une identité de séquence, pour au moins 80 %, à la partie codante (résidus n° 1 à 1152) de la SEQ ID N° 1.

20. Acide nucléique selon la revendication 19, qui comprend une identité de séquence, pour au moins 95 %, à la partie codante (résidus n° 1 à 1152) de la SEQ ID N° 1.

21. Acide nucléique selon la revendication 20, qui comprend la partie codante (résidus n° 1 à 1152) de la SEQ ID N° 1.

22. Composé liant, comprenant un site de liaison à l'antigène provenant d'un anticorps qui se lie spécifiquement à un polypeptide comprenant la séquence d'acides aminés mature (résidus n° 1 à 361) dérivée de la SEQ ID N° 2.

23. Composé liant, comprenant un site de liaison à l'antigène provenant d'un anticorps qui se lie spécifiquement à un polypeptide consistant en la séquence d'acides aminés mature (résidus n° 1 à 361) dérivée de la SEQ ID N° 2.

24. Composé liant, selon la revendication 22 ou 23, comprenant un fragment d'anticorps choisi parmi un fragment Fv, un fragment Fab et un fragment Fab2.

25. Composé liant, selon la revendication 22 ou 23, lequel composé liant est marqué de manière à pouvoir être détecté.

26. Formulation pharmaceutique qui comprend :
a) un composé liant selon la revendication 22 ou 23, et
b) un véhicule ou diluant pharmacologiquement admissible,
dans laquelle le composé liant est un anticorps doté d'une activité d'agoniste ou d'antagoniste.

27. Procédé de production *in vitro* d'un complexe antigène-anticorps, comprenant les étapes suivantes :
a) mettre un polypeptide selon la revendication 1 en contact avec un composé liant selon la revendication 22 ou 23, et
b) laisser ledit complexe se former.

28. Complexe comprenant :
a) un polypeptide selon la revendication 1, et
b) un composé liant selon la revendication 22 ou 23.

29. Composé liant selon la revendication 22 ou 23, pour lequel l'anticorps est un anticorps chimérique.

30. Composé liant selon la revendication 22 ou 23, pour lequel l'anticorps est un anticorps humanisé.

31. Composé liant selon la revendication 22 ou 23, pour lequel l'anticorps est un anticorps humain.

32. Utilisation d'un composé liant selon la revendication 22 ou 23, dans la fabrication d'un médicament, lequel composé liant est un anticorps doté d'une activité d'agoniste ou d'antagoniste.

33. Composition comprenant un composé liant selon la revendication 22 ou 23, destinée à servir de médicament, dans laquelle le composé liant est un anticorps doté d'une activité d'agoniste ou d'antagoniste.


<110> Schering Corporation
<120> Mammalian Receptor Proteins; Related Reagent and Methods
<130> DX0992 PCT
<140>
   <141>
<150> US 09/322,913
   <151> 1999-06-01
<160> 13
<170> PatentIn Ver. 2.0
<210> 1
   <211> 1155
   <212> DNA
   <213> primate
<220>
   <221> CDS
   <222> (1)..(1152)
<220>
   <221> mat peptide
   <222> (70)..(1152)
<400> 1
   atg aat cag gtc act att caa tgg gat gca gta ata gcc ctt tac ata    48
   Met Asn Gln Val Thr Ile Gln Trp Asp Ala Val Ile Ala Leu Tyr Ile
               -20                     -15                 -10
   ctc ttc agc tgg tgt cat gga gga att aca aat ata aac tgc tct ggc    96
   Leu Phe Ser Trp Cys His Gly Gly Ile Thr Asn Ile Asn Cys Ser Gly
           -5                  -1   1               5
   cac atc tgg gta gaa cca gcc aca att ttt aag atg ggt atg aat atc    144
   His Ile Trp Val Glu Pro Ala Thr Ile Phe Lys Met Gly Met Asn Ile
    10                  15                  20                  25
   tct ata tat tgc caa gca gca att aag aac tgc caa cca agg aaa ctt    192
   Ser Ile Tyr Cys Gln Ala Ala Ile Lys Asn Cys Gln Pro Arg Lys Leu
                       30                   35                   40
   cat ttt tat aaa aat ggc atc aaa gaa aga ttt caa atc aca agg att   240
   His Phe Tyr Lys Asn Gly Ile Lys Glu Arg Phe Gln Ile Thr Arg Ile
                45                  50                  55
   aat aaa aca aca gct cgg ctt tgg tat aaa aac ttt ctg gaa cca cat   288
   Asn Lys Thr Thr Ala Arg Leu Trp Tyr Lys Asn Phe Leu Glu Pro His
            60                  65                  70
   gct tct atg tac tgc act gct gaa tgt ccc aaa cat ttt caa gag aca   336
   Ala Ser Met Tyr Cys Thr Ala Glu Cys Pro Lys His Phe Gln Glu Thr
        75                  80                  85
   ctg ata tgt gga aaa gac att tct tct gga tat ccg cca gat att cct   384
   Leu Ile Cys Gly Lys Asp Ile Ser Ser Gly Tyr Pro Pro Asp Ile Pro
    90                  95                 100                 105
   gat gaa gta acc tgt gtc att tat gaa tat tca ggc aac atg act tgc   432
   Asp Glu Val Thr Cys Val Ile Tyr Glu Tyr Ser Gly Asn Met Thr Cys
                   110                 115                 120
   acc tgg aat gct ggg aag ctc acc tac ata gac aca aaa tac gtg gta   480
   Thr Trp Asn Ala Gly Lys Leu Thr Tyr Ile Asp Thr Lys Tyr Val Val
               125                 130                 135
   cat gtg aag agt tta gag aca gaa gaa gag caa cag tat ctc acc tca   528
   His Val Lys Ser Leu Glu Thr Glu Glu Glu Gln Gln Tyr Leu Thr Ser
           140                 145                 150
   agc tat att aac atc tcc act gat tca tta caa ggc ggc aag aag tac   576
   Ser Tyr Ile Asn Ile Ser Thr Asp Ser Leu Gln Gly Gly Lys Lys Tyr
       155                 160                 165
   ttg gtt tgg gtc caa gca gca aac gca cta ggc atg gaa gag tca aaa   624
   Leu Val Trp Val Gln Ala Ala Asn Ala Leu Gly Met Glu Glu Ser Lys
   170                 175                 180                 185
   caa ctg caa att cac ctg gat gat ata gtg ata cct tct gca gcc gtc   672
   Gln Leu Gln Ile His Leu Asp Asp Ile Val Ile Pro Ser Ala Ala Val
                   190                 195                 200
   att tcc agg gct gag act ata aat gct aca gtg ccc aag acc ata att   720
   Ile Ser Arg Ala Glu Thr Ile Asn Ala Thr Val Pro Lys Thr Ile Ile
               205                 210                 215
   tat tgg gat agt caa aca aca att gaa aag gtt tcc tgt gaa atg aga   768
   Tyr Trp Asp Ser Gln Thr Thr Ile Glu Lys Val Ser Cys Glu Met Arg
           220                 225                 230
   tac aag gct aca aca aac caa act tgg aat gtt aaa gaa ttt gac acc   816
   Tyr Lys Ala Thr Thr Asn Gln Thr Trp Asn Val Lys Glu Phe Asp Thr
       235                 240                 245
   aat ttt aca tat gtg caa cag tca gaa ttc tac ttg gag cca aac att   864
   Asn Phe Thr Tyr Val Gln Gln Ser Glu Phe Tyr Leu Glu Pro Asn Ile
   250                 255                 260                 265
   aag tac gta ttt caa gtg aga tgt caa gaa aca ggc aaa agg tac tgg   912
   Lys Tyr Val Phe Gln Val Arg Cys Gln Glu Thr Gly Lys Arg Tyr Trp
                   270                 275                 280
   cag cct tgg agt tca ccg ttt ttt cat aaa aca cct gaa aca gtt ccc      960
   Gln Pro Trp Ser Ser Pro Phe Phe His Lys Thr Pro Glu Thr Val Pro
               285                 290                 295
   cag gtc aca tca aaa gca ttc caa cat gac aca tgg aat tct ggg cta     1008
   Gln Val Thr Ser Lys Ala Phe Gln His Asp Thr Trp Asn Ser Gly Leu
           300                 305                 310
   aca gtt gct tcc atc tct aca ggg cac ctt act tct gac aac aga gga     1056
   Thr Val Ala Ser Ile Ser Thr Gly His Leu Thr Ser Asp Asn Arg Gly
       315                 320                 325
   gac att gga ctt tta ttg gga atg atc gtc ttt gct gtt atg ttg tca     1104
   Asp Ile Gly Leu Leu Leu Gly Met Ile Val Phe Ala Val Met Leu Ser
   330                 335                 340                 345
   att ctt tct ttg att ggg ata ttt aac aga tca ttc ccg aac tgg gat     1152
   Ile Leu Ser Leu Ile Gly Ile Phe Asn Arg Ser Phe Pro Asn Trp Asp
                   350                 355                 360
   taa                                                                 1155
<210> 2
   <211> 384
   <212> PRT
   <213> primate
<400> 2
   Met Asn Gln Val Thr Ile Gln Trp Asp Ala Val Ile Ala Leu Tyr Ile
               -20                 -15                 -10
   Leu Phe Ser Trp Cys His Gly Gly Ile Thr Asn Ile Asn Cys Ser Gly
           -5              -1  1               5
   His Ile Trp Val Glu Pro Ala Thr Ile Phe Lys Met Gly Met Asn Ile
   10                      15                  20                  25
   Ser Ile Tyr Cys Gln Ala Ala Ile Lys Asn Cys Gln Pro Arg Lys Leu
                   30                  35                  40
   His Phe Tyr Lys Asn Gly Ile Lys Glu Arg Phe Gln Ile Thr Arg Ile
               45                  50                  55
   Asn Lys Thr Thr Ala Arg Leu Trp Tyr Lys Asn Phe Leu Glu Pro His
           60                  65                  70
   Ala Ser Met Tyr Cys Thr Ala Glu Cys Pro Lys His Phe Gln Glu Thr
       75                  80                  85
   Leu Ile Cys Gly Lys Asp Ile Ser Ser Gly Tyr Pro Pro Asp Ile Pro
   90                  95                  100                 105
   Asp Glu Val Thr Cys Val Ile Tyr Glu Tyr Ser Gly Asn Met Thr Cys
                   110                 115                 120
   Thr Trp Asn Ala Gly Lys Leu Thr Tyr Ile Asp Thr Lys Tyr Val Val
               125                 130                 135
   His Val Lys Ser Leu Glu Thr Glu Glu Glu Gln Gln Tyr Leu Thr Ser
   140 145 150
   Ser Tyr Ile Asn Ile Ser Thr Asp Ser Leu Gln Gly Gly Lys Lys Tyr
   155 160 165
   Leu Val Trp Val Gln Ala Ala Asn Ala Leu Gly Met Glu Glu Ser Lys
   170 175 180 185
   Gln Leu Gln Ile His Leu Asp Asp Ile Val Ile Pro Ser Ala Ala Val
   190 195 200
   Ile Ser Arg Ala Glu Thr Ile Asn Ala Thr Val Pro Lys Thr Ile Ile
   205 210 215
   Tyr Trp Asp Ser Gln Thr Thr Ile Glu Lys Val Ser Cys Glu Met Arg
   220 225 230
   Tyr Lys Ala Thr Thr Asn Gln Thr Trp Asn Val Lys Glu Phe Asp Thr
   235 240 245
   Asn Phe Thr Tyr Val Gln Gln Ser Glu Phe Tyr Leu Glu Pro Asn Ile
   250 255 260 265
   Lys Tyr Val Phe Gln Val Arg Cys Gln Glu Thr Gly Lys Arg Tyr Trp
   270 275 280
   Gln Pro Trp Ser Ser Pro Phe Phe His Lys Thr Pro Glu Thr Val Pro
   285 290 295
   Gln Val Thr Ser Lys Ala Phe Gln His Asp Thr Trp Asn Ser Gly Leu
   300 305 310
   Thr Val Ala Ser Ile Ser Thr Gly His Leu Thr Ser Asp Asn Arg Gly
   315 320 325
   Asp Ile Gly Leu Leu Leu Gly Met Ile Val Phe Ala Val Met Leu Ser
   330 335 340 345
   Ile Leu Ser Leu Ile Gly Ile Phe Asn Arg Ser Phe Pro Asn Trp Asp
   350 355 360
<210> 3
   <211> 1152
   <212> DNA
   <213> primate
<400> 3
   atgaaycarg tnacnathca rtgggaygcn gtnathgcny tntayathyt nttywsntgg 60
   tgycayggng gnathacnaa yathaaytgy wsnggncaya thtgggtnga rccngcnacn 120
   athttyaara tgggnatgaa yathwsnath taytgycarg cngcnathaa raaytgycar 180
   ccnmgnaary tncayttyta yaaraayggn athaargarm gnttycarat hacnmgnath 240
   aayaaracna cngcnmgnyt ntggtayaar aayttyytng arccncaygc nwsnatgtay 300
   tgyacngcng artgyccnaa rcayttycar garacnytna thtgyggnaa rgayathwsn 360
   wsnggntayc cnccngayat hccngaygar gtnacntgyg tnathtayga rtaywsnggn 420
   aayatgacnt gyacntggaa ygcnggnaar ytnacntaya thgayacnaa rtaygtngtn 480
   caygtnaarw snytngarac ngargargar carcartayy tnacnwsnws ntayathaay 540
   athwsnacng aywsnytnca rggnggnaar aartayytng tntgggtnca rgcngcnaay 600
   gcnytnggna tggargarws naarcarytn carathcayy tngaygayat hgtnathccn 660
   wsngcngcng tnathwsnmg ngcngaracn athaaygcna cngtnccnaa racnathath 720
   taytgggayw sncaracnac nathgaraar gtnwsntgyg aratgmgnta yaargcnacn 780
   acnaaycara cntggaaygt naargartty gayacnaayt tyacntaygt ncarcarwsn 840
   garttytayy tngarccnaa yathaartay gtnttycarg tnmgntgyca rgaracnggn 900
   aarmgntayt ggcarccntg gwsnwsnccn ttyttycaya aracnccnga racngtnccn 960
   cargtnacnw snaargcntt ycarcaygay acntggaayw snggnytnac ngtngcnwsn 1020
   athwsnacng gncayytnac nwsngayaay mgnggngaya thggnytnyt nytnggnatg 1080
   athgtnttyg cngtnatgyt nwsnathytn wsnytnathg gnathttyaa ymgnwsntty 1140
   ccnaaytggg ay                                                     1152
<210> 4
   <211> 410
   <212> PRT
   <213> primate
<400> 4.
   Met Pro Ala Gly Arg Arg Gly Pro Ala Ala Gln Ser Ala Arg Arg Pro
    1               5                  10                  15
   Pro Pro Leu Leu Pro Leu Leu Leu Leu Leu Cys Val Leu Gly Ala Pro
               20                  25                  30
   Arg Ala Gly Ser Gly Ala His Thr Ala Val Ile Ser Pro Gln Asp Pro
           35                  40                  45
   Thr Leu Leu Ile Gly Ser Ser Leu Leu Ala Thr Cys Ser Val His Gly
       50                  55                  60
   Asp Pro Pro Gly Ala Thr Ala Glu Gly Leu Tyr Trp Thr Leu Asn Gly
   65                  70                  75                  80
   Arg Arg Leu Pro Pro Glu Leu Ser Arg Val Leu Asn Ala Ser Thr Leu
                   85                  90                  95
   Ala Leu Ala Leu Ala Asn Leu Asn Gly Ser Arg Gln Arg Ser Gly Asp
               100                 105                 110
   Asn Leu Val Cys His Ala Arg Asp Gly Ser Ile Leu Ala Gly Ser Cys
           115                 120                 125
   Leu Tyr Val Gly Leu Pro Pro Glu Lys Pro Val Asn Ile Ser Cys Trp
       130                 135                 140
   Ser Lys Asn Met Lys Asp Leu Thr Cys Arg Trp Thr Pro Gly Ala His
   145                 150                 155                 160
   Gly Glu Thr Phe Leu His Thr Asn Tyr Ser Leu Lys Tyr Lys Leu Arg
                   165                 170                 175
   Trp Tyr Gly Gln Asp Asn Thr Cys Glu Glu Tyr His Thr Val Gly Pro
               180                 185                 190
   His Ser Cys His Ile Pro Lys Asp Leu Ala Leu Phe Thr Pro Tyr Glu
           195                 200                 205
   Ile Trp Val Glu Ala Thr Asn Arg Leu Gly Ser Ala Arg Ser Asp Val
       210                 215                 220
   Leu Thr Leu Asp Ile Leu Asp Val Val Thr Thr Asp Pro Pro Pro Asp
   225                 230                 235                 240
   Val His Val Ser Arg Val Gly Gly Leu Glu Asp Gln Leu Ser Val Arg
                   245                 250                 255
   Trp Val Ser Pro Pro Ala Leu Lys Asp Phe Leu Phe Gln Ala Lys Tyr
               260                 265                 270
   Gln Ile Arg Tyr Arg Val Glu Asp Ser Val Asp Trp Lys Val Val Asp
           275                 280                 285
   Asp Val Ser Asn Gln Thr Ser Cys Arg Leu Ala Gly Leu Lys Pro Gly
       290                 295                 300
   Thr Val Tyr Phe Val Gln Val Arg Cys Asn Pro Phe Gly Ile Tyr Gly
   305                 310                 315                 320
   Ser Lys Lys Ala Gly Ile Trp Ser Glu Trp Ser His Pro Thr Ala Ala
                   325                 330                 335
   Ser Thr Pro Arg Ser Glu Arg Pro Gly Pro Gly Gly Gly Ala Cys Glu
               340                 345                 350
   Pro Arg Gly Gly Glu Pro Ser Ser Gly Pro Val Arg Arg Glu Leu Lys
           355                 360                 365
   Gln Phe Leu Gly Trp Leu Lys Lys His Ala Tyr Cys Ser Asn Leu Ser
       370                 375                 380
   Phe Arg Leu Tyr Asp Gln Trp Arg Ala Trp Met Gln Lys Ser His Lys
   385                 390                 395                 400
   Thr Arg Asn Gln Val Leu Pro Asp Lys Leu
                   405                 410
<210> 5
   <211> 407
   <212> PRT
   <213> rodent
<400> 5
   Arg Pro Leu Ser Ser Leu Trp Ser Pro Leu Leu Leu Cys Val Leu Gly
   1               5                   10                  15
   Val Pro Arg Gly Gly Ser Gly Ala His Thr Ala Val Ile Ser Pro Gln
               20                  25                  30
   Asp Pro Thr Leu Leu Ile Gly Ser Ser Leu Gln Ala Thr Cys Ser Ile
           35                  40                  45
   His Gly Asp Thr Pro Gly Ala Thr Ala Glu Gly Leu Tyr Trp Thr Leu
       50                  55                  60
   Asn Gly Arg Arg Leu Pro Ser Leu Ser Arg Leu Leu Asn Thr Ser Thr
   65                  70                  75                  80
   Leu Ala Leu Ala Leu Ala Asn Leu Asn Gly Ser Arg Gln Gln Ser Gly
                   85                  90                  95
   Asp Asn Leu Val Cys His Ala Arg Asp Gly Ser Ile Leu Ala Gly Ser
               100                 105                 110
   Cys Leu Tyr Val Gly Leu Pro Pro Glu Lys Pro Phe Asn Ile Ser Cys
           115                 120                 125
   Trp Ser Arg Asn Met Lys Asp Leu Thr Cys Arg Trp Thr Pro Gly Ala
       130                 135                 140
   His Gly Glu Thr Phe Leu His Thr Asn Tyr Ser Leu Lys Tyr Lys Leu
   145                 150                 155                 160
   Arg Trp Tyr Gly Gln Asp Asn Thr Cys Glu Glu Tyr His Thr Val Gly
                   165                 170                 175
   Pro His Ser Cys His Ile Pro Lys Asp Leu Ala Leu Phe Thr Pro Tyr
               180                 185                 190
   Glu Ile Trp Val Glu Ala Thr Asn Arg Leu Gly Ser Ala Arg Ser Asp
           195                 200                 205
   Val Leu Thr Leu Asp Val Leu Asp Val Val Thr Thr Asp Pro Pro Pro
       210                 215                 220
   Asp Val His Val Ser Arg Val Gly Gly Leu Glu Asp Gln Leu Ser Val
   225                 230                 235                 240
   Arg Trp Val Ser Pro Pro Ala Leu Lys Asp Phe Leu Phe Gln Ala Lys
                   245                 250                 255
   Tyr Gln Ile Arg Tyr Arg Val Glu Asp Ser Val Asp Trp Lys Val Val
               260                 265                 270
   Asp Asp Val Ser Asn Gln Thr Ser Cys Arg Leu Ala Gly Leu Lys Pro
           275                 280                 285
   Gly Thr Val Tyr Phe Val Gln Val Arg Cys Asn Pro Phe Gly Ile Tyr
       290                 295                 300
   Gly Ser Lys Lys Ala Gly Ile Trp Ser Glu Trp Ser His Pro Thr Ala
   305                 310                 315                 320
   Ala Ser Thr Pro Arg Ser Glu Arg Pro Gly Pro Gly Gly Gly Val Cys
                   325                 330                 335
   Glu Pro Arg Gly Gly Glu Pro Ser Ser Gly Pro Val Arg Arg Glu Leu
   340 345 350
   Lys Gln Phe Leu Gly Trp Leu Lys Lys His Ala Tyr Cys Ser Asn Leu
   355 360 365
   Ser Phe Arg Leu Tyr Asp Gln Trp Arg Ala Trp Met Gln Lys Ser His
   370 375 380
   Lys Thr Arg Asn Gln Asp Glu Gly Ile Leu Pro Ser Gly Arg Arg Gly
   385 390 395 400
   Ala Ala Arg Gly Pro Ala Gly
   405
<210> 6
   <211> 328
   <212> PRT
   <213> primate
<400> 6
   Met Cys His Gln Gln Leu Val Ile Ser Trp Phe Ser Leu Val Phe Leu
    1               5                  10                  15
   Ala Ser Pro Leu Val Ala Ile Trp Glu Leu Lys Lys Asp Val Tyr Val
               20                  25                  30
   Val Glu Leu Asp Trp Tyr Pro Asp Ala Pro Gly Glu Met Val Val Leu
           35                  40                  45
   Thr Cys Asp Thr Pro Glu Glu Asp Gly Ile Thr Trp Thr Leu Asp Gln
       50                  55                  60
   Ser Ser Glu Val Leu Gly Ser Gly Lys Thr Leu Thr Ile Gln Val Lys
   65                  70                  75                  80
   Glu Phe Gly Asp Ala Gly Gln Tyr Thr Cys His Lys Gly Gly Glu Val
                   85                  90                  95
   Leu Ser His Ser Leu Leu Leu Leu His Lys Lys Glu Asp Gly Ile Trp
               100                 105                 110
   Ser Thr Asp Ile Leu Lys Asp Gln Lys Glu Pro Lys Asn Lys Thr Phe
           115                 120                 125
   Leu Arg Cys Glu Ala Lys Asn Tyr Ser Gly Arg Phe Thr Cys Trp Trp
       130                 135                 140
   Leu Thr Thr Ile Ser Thr Asp Leu Thr Phe Ser Val Lys Ser Ser Arg
   145                 150                 155                 160
   Gly Ser Ser Asp Pro Gln Gly Val Thr Cys Gly Ala Ala Thr Leu Ser
                   165                 170                 175
   Ala Glu Arg Val Arg Gly Asp Asn Lys Glu Tyr Glu Tyr Ser Val Glu
               180                 185                 190
   Cys Gln Glu Asp Ser Ala Cys Pro Ala Ala Glu Glu Ser Leu Pro Ile
           195                 200                 205
   Glu Val Met Val Asp Ala Val His Lys Leu Lys Tyr Glu Asn Tyr Thr
               210                    215                    220
   Ser Ser Phe Phe Ile Arg Asp Ile Ile Lys Pro Asp Pro Pro Lys Asn
   225                 230                     235                 240
   Leu Gln Leu Lys Pro Leu Lys Asn Ser Arg Gln Val Glu Val Ser Trp
                   245                     250                 255
   Glu Tyr Pro Asp Thr Trp Ser Thr Pro His Ser Tyr Phe Ser Leu Thr
               260                 265                 270
   Phe Cys Val Gln Val Gln Gly Lys Ser Lys Arg Glu Lys Lys Asp Arg
           275                 280                 285
   Val Phe Thr Asp Lys Thr Ser Ala Thr Val Ile Cys Arg Lys Asn Ala
       290                 295                 300
   Ser Ile Ser Val Arg Ala Gln Asp Arg Tyr Tyr Ser Ser Ser Trp Ser
   305                 310                 315                 320
   Glu Trp Ala Ser Val Pro Cys Ser
                   325
<210> 7
   <211> 335
   <212> PRT
   <213> rodent
<400> 7
   Met Cys Pro Gln Lys Leu Thr Ile Ser Trp Phe Ala Ile Val Leu Leu
    1               5                  10                  15
   Val Ser Pro Leu Met Ala Met Trp Glu Leu Glu Lys Asp Val Tyr Val
               20                  25                  30
   Val Glu Val Asp Trp Thr Pro Asp Ala Pro Gly Glu Thr Val Asn Leu
           35                  40                  45
   Thr Cys Asp Thr Pro Glu Glu Asp Asp Ile Thr Trp Thr Ser Asp Gln
       50                  55                  60
   Arg His Gly Val Ile Gly Ser Gly Lys Thr Leu Thr Ile Thr Val Lys
   65                  70                  75                  80
   Glu Phe Leu Asp Ala Gly Gln Tyr Thr Cys His Lys Gly Gly Glu Thr
                   85                  90                  95
   Leu Ser His Ser His Leu Leu Leu His Lys Lys Glu Asn Gly Ile Trp
               100                 105                 110
   Ser Thr Glu Ile Leu Lys Asn Phe Lys Asn Lys Thr Phe Leu Lys Cys
           115                 120                 125
   Glu Ala Pro Asn Tyr Ser Gly Arg Phe Thr Cys Ser Trp Leu Val Gln
       130                 135                 140
   Arg Asn Met Asp Leu Lys Phe Asn Ile Lys Ser Ser Ser Ser Ser Pro
   145                 150                 155                 160
   Asp Ser Arg Ala Val Thr Cys Gly Met Ala Ser Leu Ser Ala Glu Lys
                           165                     170                     175
   Val Thr Leu Asp Gln Arg Asp Tyr Glu Lys Tyr Ser Val Ser Cys Gln
               180                     185                     190
   Glu Asp Val Thr Cys Pro Thr Ala Glu Glu Thr Leu Pro Ile Glu Leu
           195                     200                     205
   Ala Leu Glu Ala Arg Gln Gln Asn Lys Tyr Glu Asn Tyr Ser Thr Ser
       210                     215                     220
   Phe Phe Ile Arg Asp Ile Ile Lys Pro Asp Pro Pro Lys Asn Leu Gln
   225                     230                     235                     240
   Met Lys Pro Leu Lys Asn Ser Gln Val Glu Val Ser Trp Glu Tyr Pro
                   245                     250                     255
   Asp Ser Trp Ser Thr Pro His Ser Tyr Phe Ser Leu Lys Phe Phe Val
               260                     265                     270
   Arg Ile Gln Arg Lys Lys Glu Lys Met Lys Glu Thr Glu Glu Gly Cys
           275                     280                     285
   Asn Gln Lys Gly Ala Phe Leu Val Glu Lys Thr Ser Thr Glu Val Gln
       290                     295                     300
   Cys Lys Gly Gly Asn Val Cys Val Gln Ala Gln Asp Arg Tyr Tyr Asn
   305                     310                     315                     320
   Ser Ser Cys Ser Lys Trp Ala Cys Val Pro Cys Arg Val Arg Ser
                   325                     330                     335
<210> 8
   <211> 228
   <212> PRT
   <213> rodent
<400> 8
   Met Ser Lys Leu Leu Phe Leu Ser Leu Ala Leu Trp Ala Ser Arg Ser
    1               5                  10                  15
   Pro Gly Tyr Thr Glu Thr Ala Leu Val Ala Leu Ser Gln Pro Arg Val
               20                  25                  30
   Gln Cys His Ala Ser Arg Tyr Pro Val Ala Val Asp Cys Ser Trp Thr
           35                  40                  45
   Pro Leu Gln Ala Pro Asn Ser Thr Arg Ser Thr Ser Phe Ile Ala Thr
       50                  55                  60
   Tyr Arg Leu Gly Val Ala Thr Gln Gln Gln Ser Gln Pro Cys Leu Gln
   65                  70                  75                  80
   Arg Ser Pro Gln Ala Ser Arg Cys Thr Ile Pro Asp Val His Leu Phe
                   85                  90                  95
   Ser Thr Val Pro Tyr Met Leu Asn Val Thr Ala Val His Pro Gly Gly
               100                 105                 110
   Ala Ser Ser Ser Leu Leu Ala Phe Val Ala Glu Arg Ile Ile Lys Pro
           115                 120                 125
   Asp Pro Pro Glu Gly Val Arg Leu Arg Thr Ala Gly Gln Arg Leu Gln
       130                 135                 140
   Val Leu Trp His Pro Pro Ala Ser Trp Pro Phe Pro Asp Ile Phe Ser
   145                 150                 155                 160
   Leu Lys Tyr Arg Leu Arg Tyr Arg Arg Arg Gly Ala Ser His Phe Arg
                   165                 170                 175
   Gln Val Gly Pro Ile Glu Ala Thr Thr Phe Thr Leu Arg Asn Ser Lys
               180                 185                 190
   Pro His Ala Lys Tyr Cys Ile Gln Val Ser Ala Gln Asp Leu Thr Asp
           195                 200                 205
   Tyr Gly Lys Pro Ser Asp Trp Ser Leu Pro Gly Gln Val Glu Ser Ala
       210                 215                 220
   Pro His Lys Pro
   225
<210> 9
   <211> 229
   <212> PRT
   <213> primate
<400> 9
<210> 10
   <211> 432
   <212> PRT
   <213> rodent
<400> 10
   Met Ser Ser Ser Cys Ser Gly Leu Thr Arg Val Leu Val Ala Val Ala
   1 5 10 15
   Thr Ala Leu Val Ser Ser Ser Ser Pro Cys Pro Gln Ala Trp Gly Pro
   20 25 30
   Pro Gly Val Gln Tyr Gly Gln Pro Gly Arg Pro Val Met Leu Cys Cys
   35 40 45
   Pro Gly Val Ser Ala Gly Thr Pro Val Ser Trp Phe Arg Asp Gly Asp
   50 55 60
   Ser Arg Leu Leu Gln Gly Pro Asp Ser Gly Leu Gly His Arg Leu Val
   65 70 75 80
   Leu Ala Gln Val Asp Ser Pro Asp Glu Gly Thr Tyr Val Cys Gln Thr
   85 90 95
   Leu Asp Gly Val Ser Gly Gly Met Val Thr Leu Lys Leu Gly Phe Pro
   100 105 110
   Pro Ala Arg Pro Glu Val Ser Cys Gln Ala Val Asp Tyr Glu Asn Phe
   115 120 125
   Ser Cys Thr Trp Ser Pro Gly Gln Val Ser Gly Leu Pro Thr Arg Tyr
   130 135 140
   Leu Thr Ser Tyr Arg Lys Lys Thr Leu Pro Gly Ala Glu Ser Gln Arg
   145 150 155 160
   Glu Ser Pro Ser Thr Gly Pro Trp Pro Cys Pro Gln Asp Pro Leu Glu
   165 170 175
   Ala Ser Arg Cys Val Val His Gly Ala Glu Phe Trp Ser Glu Tyr Arg
   180 185 190
   Ile Asn Val Thr Glu Val Asn Ser Leu Gly Ala Ser Thr Cys Leu Leu
   195 200 205
   Asp Val Arg Leu Gln Ser Ile Leu Arg Pro Asp Pro Pro Gln Gly Leu
   210 215 220
   Arg Val Glu Ser Val Pro Gly Tyr Pro Arg Arg Leu His Ala Ser Trp
   225 230 235 240
   Thr Tyr Pro Ala Ser Trp Arg Arg Gln Pro His Phe Leu Leu Lys Phe
   245 250 255
   Arg Leu Gln Tyr Arg Pro Ala Gln His Pro Ala Trp Ser Thr Val Glu
   260 265 270
   Pro Ile Gly Leu Glu Glu Val Ile Thr Asp Thr Val Ala Gly Leu Pro
   275 280 285
   His Ala Val Arg Val Ser Ala Arg Asp Phe Leu Asp Ala Gly Thr Trp
   290 295 300
   Ser Ala Trp Ser Pro Glu Ala Trp Gly Thr Pro Ser Thr Gly Leu Leu
   305 310 315 320
   Gln Asp Glu Ile Pro Asp Trp Ser Gln Gly His Gly Gln Gln Leu Glu
   325 330 335
   Ala Val Val Ala Gln Glu Asp Ser Leu Ala Pro Ala Arg Pro Ser Leu
   340 345 350
   Gln Pro Asp Pro Arg Pro Leu Asp His Arg Asp Pro Leu Glu Gln Val
   355 360 365
   Ala Val Leu Ala Ser Leu Gly Ile Phe Ser Cys Leu Gly Leu Ala Val
   370 375 380
   Gly Ala Leu Ala Leu Gly Leu Trp Leu Arg Leu Arg Arg Ser Gly Lys
   385 390 395 400
   Glu Gly Pro Gln Lys Pro Gly Leu Leu Ala Pro Met Ile Pro Val Glu
   405 410 415
   Lys Leu Pro Gly Ile Pro Asn Leu Gln Arg Thr Pro Glu Asn Phe Ser
   420 425 430
<210> 11
   <211> 422
   <212> PRT
   <213> primate
<400> 11
<210> 12
   <211> 468
   <212> PRT
   <213> primate
<400> 12
   Met Leu Ala Val Gly Cys Ala Leu Leu Ala Ala Leu Leu Ala Ala Pro
    1               5                  10                  15
   Gly Ala Ala Leu Ala Pro Arg Arg Cys Pro Ala Gln Glu Val Ala Arg
               20                  25                  30
   Gly Val Leu Thr Ser Leu Pro Gly Asp Ser Val Thr Leu Thr Cys Pro
           35                  40                  45
   Gly Val Glu Pro Glu Asp Asn Ala Thr Val His Trp Val Leu Arg Lys
       50                  55                  60
   Pro Ala Ala Gly Ser His Pro Ser Arg Trp Ala Gly Met Gly Arg Arg
   65                  70                  75                  80
   Leu Leu Leu Arg Ser Val Gln Leu His Asp Ser Gly Asn Tyr Ser Cys
                   85                  90                  95
   Tyr Arg Ala Gly Arg Pro Ala Gly Thr Val His Leu Leu Val Asp Val
               100                 105                 110
   Pro Pro Glu Glu Pro Gln Leu Ser Cys Phe Arg Lys Ser Pro Leu Ser
           115                 120                 125
   Asn Val Val Cys Glu Trp Gly Pro Arg Ser Thr Pro Ser Leu Thr Thr
       130                 135                 140
   Lys Ala Val Leu Leu Val Arg Lys Phe Gln Asn Ser Pro Ala Glu Asp
   145                 150                 155                 160
   Phe Gln Glu Pro Cys Gln Tyr Ser Gln Glu Ser Gln Lys Phe Ser Cys
                   165                 170                 175
   Gln Leu Ala Val Pro Glu Gly Asp Ser Ser Phe Tyr Ile Val Ser Met
               180                 185                 190
   Cys Val Ala Ser Ser Val Gly Ser Lys Phe Ser Lys Thr Gln Thr Phe
           195                 200                 205
   Gln Gly Cys Gly Ile Leu Gln Pro Asp Pro Pro Ala Asn Ile Thr Val
       210                 215                 220
   Thr Ala Val Ala Arg Asn Pro Arg Trp Leu Ser Val Thr Trp Gln Asp
   225                 230                 235                 240
   Pro His Ser Trp Asn Ser Ser Phe Tyr Arg Leu Arg Phe Glu Leu Arg
   245 250 255
   Tyr Arg Ala Glu Arg Ser Lys Thr Phe Thr Thr Trp Met Val Lys Asp
   260 265 270
   Leu Gln His His Cys Val Ile His Asp Ala Trp Ser Gly Leu Arg His
   275 280 285
   Val Val Gln Leu Arg Ala Gln Glu Glu Phe Gly Gln Gly Glu Trp Ser
   290 295 300
   Glu Trp Ser Pro Glu Ala Met Gly Thr Pro Trp Thr Glu Ser Arg Ser
   305 310 315 320
   Pro Pro Ala Glu Asn Glu Val Ser Thr Pro Met Gln Ala Leu Thr Thr
   325 330 335
   Asn Lys Asp Asp Asp Asn Ile Leu Phe Arg Asp Ser Ala Asn Ala Thr
   340 345 350
   Ser Leu Pro Val Gln Asp Ser Ser Ser Val Pro Leu Pro Thr Phe Leu
   355 360 365
   Val Ala Gly Gly Ser Leu Ala Phe Gly Thr Leu Leu Cys Ile Ala Ile
   370 375 380
   Val Leu Arg Phe Lys Lys Thr Trp Lys Leu Arg Ala Leu Lys Glu Gly
   385 390 395 400
   Lys Thr Ser Met His Pro Pro Tyr Ser Leu Gly Gln Leu Val Pro Glu
   405 410 415
   Arg Pro Arg Pro Thr Pro Val Leu Val Pro Leu Ile Ser Pro Pro Val
   420 425 430
   Ser Pro Ser Ser Leu Gly Ser Asp Asn Thr Ser Ser His Asn Arg Pro
   435 440 445
   Asp Ala Arg Asp Pro Arg Ser Pro Tyr Asp Ile Ser Asn Thr Asp Tyr
   450 455 460
   Phe Phe Pro Arg
   465
<210> 13
   <211> 460
   <212> PRT
   <213> rodent
<400> 13
   Met Leu Thr Val Gly Cys Thr Leu Leu Val Ala Leu Leu Ala Ala Pro
    1               5                  10                  15
   Ala Val Ala Leu Val Leu Gly Ser Cys Arg Ala Leu Glu Val Ala Asn
               20                  25                  30
   Gly Thr Val Thr Ser Leu Pro Gly Ala Thr Val Thr Leu Ile Cys Pro
           35                  40                  45
   Gly Lys Glu Ala Ala Gly Asn Val Thr Ile His Trp Val Tyr Ser Gly
   50 55 60
   Ser Gln Asn Arg Glu Trp Thr Thr Thr Gly Asn Thr Leu Val Leu Arg
   65 70 75 80
   Asp Val Gln Leu Ser Asp Thr Gly Asp Tyr Leu Cys Ser Leu Asn Asp
   85 90 95
   His Leu Val Gly Thr Val Pro Leu Leu Val Asp Val Pro Pro Glu Glu
   100 105 110
   Pro Lys Leu Ser Cys Phe Arg Lys Asn Pro Leu Val Asn Ala Ile Cys
   115 120 125
   Glu Trp Arg Pro Ser Ser Thr Pro Ser Pro Thr Thr Lys Ala Val Leu
   130 135 140
   Phe Ala Lys Lys Ile Asn Thr Thr Asn Gly Lys Ser Asp Phe Gln Val
   145 150 155 160
   Pro Cys Gln Tyr Ser Gln Gln Leu Lys Ser Phe Ser Cys Gln Val Glu
   165 170 175
   Ile Leu Glu Gly Asp Lys Val Tyr His Ile Val Ser Leu Cys Val Ala
   180 185 190
   Asn Ser Val Gly Ser Lys Ser Ser His Asn Glu Ala Phe His Ser Leu
   195 200 205
   Lys Met Val Gln Pro Asp Pro Pro Ala Asn Leu Val Val Ser Ala Ile
   210 215 220
   Pro Gly Arg Pro Arg Trp Leu Lys Val Ser Trp Gln His Pro Glu Thr
   225 230 235 240
   Trp Asp Pro Ser Tyr Tyr Leu Leu Gln Phe Gln Leu Arg Tyr Arg Pro
   245 250 255
   Val Trp Ser Lys Glu Phe Thr Val Leu Leu Leu Pro Val Ala Gln Tyr
   260 265 270
   Gln Cys Val Ile His Asp Ala Leu Arg Gly Val Lys His Val Val Gln
   275 280 285
   Val Arg Gly Lys Glu Glu Leu Asp Leu Gly Gln Trp Ser Glu Trp Ser
   290 295 300
   Pro Glu Val Thr Gly Thr Pro Trp Ile Ala Glu Pro Arg Thr Thr Pro
   305 310 315 320
   Ala Gly Ile Leu Trp Asn Pro Thr Gln Val Ser Val Glu Asp Ser Ala
   325 330 335
   Asn His Glu Asp Gln Tyr Glu Ser Ser Thr Glu Ala Thr Ser Val Leu
   340 345 350
   Ala Pro Val Gln Glu Ser Ser Ser Met Ser Leu Pro Thr Phe Leu Val
   355 360 365
   Ala Gly Gly Ser Leu Ala Phe Gly Leu Leu Leu Cys Val Phe Ile Ile
   370 375 380
   Leu Arg Leu Lys Gln Lys Trp Lys Ser Glu Ala Glu Lys Glu Ser Lys
   385 390 395 400
   Thr Thr Ser Pro Pro Pro Pro Pro Tyr Ser Leu Gly Pro Leu Lys Pro
   405 410 415
   Thr Phe Leu Leu Val Pro Leu Leu Thr Pro His Ser Ser Gly Ser Asp
   420 425 430
   Asn Thr Val Asn His Ser Cys Leu Gly Val Arg Asp Ala Gln Ser Pro
   435 440 445
   Tyr Asp Asn Ser Asn Arg Asp Tyr Leu Phe Pro Arg
   450 455 460